# EUROPEAN PATENT SPECIFICATION

(11) **EP 2 215 168 B1**
(45) Date of publication and mention of the grant of the patent: **30.07.2014**
(21) Application number: 08849324.2
(22) Date of filing: 12.11.2008
(51) Int. Cl.: C09D 5/00, A61K 9/00

(54) **ANTI-FOULING COMPOSITION COMPRISING AN AEROGEL**
ANTIFOULINGMITTEL MIT AEROGEL
COMPOSITION ANTISALISSURE COMPRENANT UN AÉROGEL

(30) Priority: 12.11.2007 DK 200701594; 12.11.2007 US 987221 P; 06.06.2008 US 59353 P; 06.06.2008 EP 08157766
(43) Date of publication of application: 11.08.2010
(73) Proprietor: CoatZyme Aps, 2800 Lyngby (DK)
(72) Inventor: TOFTE JESPERSEN, Henrik, DK-4070 Kirke Hyllinge (DK); ALLERMANN, Knud, DK-2960 Rungsted Kyst (DK); SCHNEIDER, Ib, DK-2820 Gentofte (DK); SCHAUMBURG, Kjeld, DK-2730 Herlev (DK)
(74) Representative: Leifert & Steffan
(86) International application number: PCT/DK2008/050272
(87) International publication number: WO 2009/062518

(56) References cited:
- EP-A- 1 825 752
- EP-A- 1 882 722
- WO-A-03/096863
- DE-A1-102004 008 931
- US-A1- 2001 026 802
- US-A1- 2002 094 318
- US-A1- 2003 213 505
- US-A1- 2007 225 515

## Description

### Background of invention

### Aerogels

Aerogel is a low-density solid-state material derived from gel in which the liquid component of the gel has been replaced with gas. The result is a low density solid with several characteristic properties such as its effectiveness as an insulator.

Aerogel was first created by Steven Kistler in 1931. Aerogels are in general produced by extracting the liquid component of a gel through supercritical drying. This allows the liquid to be slowly drawn off without causing the solid matrix in the gel to collapse from capillary action, as would happen with conventional evaporation. The first aerogels were produced from silica gels. Aerogels based on alumina, chromia and tin oxide have also been described. Carbon aerogels were developed in the early 1990s.

### Anti-fouling agent

In order to minimize the impacts of foulers, many underwater structures are protected by antifouling coatings. Coatings, however, have been found to be toxic to marine organisms. For example, extremely low concentrations of tributyltin moiety (TBT), the mostly commonly used anti-fouling agent, cause defective shell growth in the oyster *Crassostrea gigas* (at a concentration of 20 ng/l) and development of male characteristics in female genitalia in the dog whelk *Nucella lapillus* (where gonocharacteristic change is initiated at 1 ng/l). The ban of organotins such as TBT and triphenyltin (TPT), and other toxic biocides in marine coatings is a severe problem for the shipping industry; it presents a major challenge for the producers of coatings to develop alternative technologies to prevent fouling on ship hulls. Safer methods of biofouling control are actively researched. Copper and derivative compounds have successfully been used either in paints or as metal sheeting (for example Muntz metal which was specifically made for this purpose), though there is still debate as to the safety of copper.

### Summary of invention

The present invention relates to an anti-fouling composition comprising one or more aerogels wherein the aerogel encapsulates one or more bioactive agents. The one or more encapsulated bioactive agents can in one preferred embodiment be released from the aerogel over time. The encapsulated bioactive agents comprises enzymes.

### Definitions

Aerogel is a low-density solid-state material derived from gel in which the liquid component of the gel has been replaced with gas.

Anti-fouling is the process of removing or inhibiting the accumulation of biofoulling.

Antifouling species: Any species exerting antifouling effect. Species such as antimicrobial species, antibacterial species, antifungal species, biocides, biorepellents, and the like. Bioactive agents and antifouling species are used interchangeable herein.

Bio-film: Habitation of microbial organisms on a solid or semi-solid surface.

Biofouling or biological fouling is the undesirable accumulation of microorganisms, plants, algae, and animals on surfaces such as submerged structures like ships' hulls. Subtilisins comprises a family of serine proteases isolated from bacillus subtilis. Anti-fouling: The effect of controlling, reducing and/or eliminating over time the number of undesirable microorganisms in a bio-film.

Coating composition: Composition for coating an object, such as a paint.

Co-factor: Additional factor required by an enzyme.

Compound: Substrate for an enzyme capable of catalysing said compound, wherein said catalysis results in the formation of an antimicrobial species comprising an antimicrobial activity.

Enzyme: Biomolecule comprising a plurality of amino acids and capable of catalysing conversion of substrates into products. The terms enzyme and precursor enzyme are used interchangably unless otherwise indicated. An enzyme is acting on a compound as defined herein when said action generates an antifouling species having antifouling activity. A precursor enzyme is any enzyme capable of providing to the enzyme, by means of degradation or otherwise, a substrate for said enzyme in the form of said compound.

Lipid or lipid composition: When used herein in connection with modification and/or coating of enzymes, lipid means a compound having a long-chain alkyl group, which is a hydrophobic group, and a hydrophilic group.

Marine organism: Any organism capable of habitating in an aqueous environment, including organisms capable of forming undesirable bio-films.

Microbial organism: Any organism belonging to the classes of prokaryotes and lower eukaryotes, including bacteria, yeasts, fungal cells and slime molds.

Oxidase: Enzyme the activity of which results in an oxidation, including an oxidation resulting in the formation of a peroxide, including hydrogenperoxide.

Paint is any liquid, liquifiable, or mastic composition which after application to a surface in a thin layer is converted to an opaque solid film.

Painting is the application of paint.

Peroxide: Product resulting from a reaction involving an oxidase.

Precursor compound: Precursor compounds are capable of being catalysed by a precursor enzyme, wherein said catalysis results in the formation of a compound capable of being catalysed by an enzyme under the generation of an antifouling species, including an antimicrobial species having an antimicrobial activity.

Secretion: Process of translocating a compound or precursor compound across the outer membrane of a microbial species. Secretion applies to compounds which remain membrane associated and to compounds which are subsequently released into an external environment.

Surface: Outer part of e.g. a microbial organism in contact with the external environment.

Xerogel: A xerogel is a solid formed from a gel by drying with unhindered shrinkage. Xerogel usually retains high porosity (25%) and enormous surface area (150-900 m²/g), along with very small pore size (1-10 nm). When solvent removal occurs under hypercritical (supercritical) conditions, the network does not shrink and a highly porous, low-density material known as an *aerogel* is produced. Heat treatment of a xerogal at elevated temperature produces viscous sintering and effectively transforms the porous gel into a dense glass.

### Detailed description of the invention

One embodiment not according to the invention relates to a composition comprising a xerogel and at least one enzyme capable of acting on a compound, wherein said action results in the formation of an antifouling species comprising an antifouling activity. The compound can form part of said composition, but it does not need to form part of said composition. The latter is the case when the compound is e.g. provided by an external environment in which the composition is exerting an antifouling effect. The composition is preferably has a self-polishing effect.

The subject-matter of the present invention is expressed in claim 1. Preferred embodiments of the present invention are described in dependent claims 2 to 17. Further embodiments in the description are not according to the invention but serve for illustrative purposes only.

The present invention in another embodiment relates to a composition comprising an aerogel and at least one enzyme capable of acting on a compound, wherein said action results in the formation of an antifouling species comprising an antifouling activity. The compound can form part of said composition, but it does not need to form part of said composition. The latter is the case when the compound is e.g. provided by an external environment in which the composition is exerting an antifouling effect. The composition is preferably has a self-polishing effect.

The composition can be a coating composition further comprising a pigment, or a hygienic composition further comprising a fragrance, or a composition as stated herein above further comprising both a pigment and a fragrance.

In another embodiment there is provided a method for preparing such compositions, including a coating composition, as well as uses of such a coating composition, including uses as described in more detail herein below.

In yet another embodiment there is provided a method for reducing marine corrosion comprising the step of coating a marine surface with a marine antifouling composition, whereby the composition forms at least one film that reduces adsorption of corrosive molecules to the surface. Also disclosed is a method wherein the composition impedes surface corrosion and intergranular corrosion.

In a still further embodiment there is provided a method for reducing marine corrosion comprising the step of coating a marine surface with a marine antifouling paint, whereby the paint forms at least one film that reduces adsorption of corrosive molecules to the surface. In yet another embodiment of the claimed invention, a method is disclosed, wherein the paint impedes surface corrosion and intergranular corrosion.

In an even further embodiment there is provided a method for limiting absorption of water by a marine surface comprising the step of coating the surface with a marine antifouling composition or marine antifouling paint, whereby the composition or paint produces a film which in turn reduces the porosity of the surface.

In still another embodiment, a method is disclosed for reducing the coefficient of drag of a marine surface comprising the step of coating the surface with a marine antifouling composition or marine antifouling paint. The invention is also directed to methods of using the marine antifouling composition or marine antifouling paint wherein surfactants capable of acting as wetting agents are produced by microorganisms in contact with the composition or paint.

Yet another embodiment is directed to a method for removing marine growth from a marine surface, comprising the step of coating the surface with a marine antifouling composition or marine antifouling paint. Another embodiment is a method of using the marine antifouling composition or marine antifouling paint wherein the marine growth is hard or soft growth.

An even further embodiment is directed to a method of using the marine antifouling composition or marine antifouling paint, wherein e.g. hydrolytic enzymes attack exudates of existing growths and causes release of hard and soft growth.

### Types of aerogels:

In one embodiment the present invention relates to silica aerogels gels or carbon aerogels. Alternatively the aerogels are based on alumina, chromia, tin and bromo oxide.

Metal-aerogel nanocomposites can be prepared by impregnating the hydrogel with solution containing ions of the suitable noble or transition metals. The impregnated hydrogel is then, in one embodiment, irradiated with gamma rays, leading to precipitation of nanoparticles of the metal. Such composites can be used as eg. catalysts, sensors, electromagnetic shielding, and in waste disposal. A prospective use of platinum-on-carbon catalysts is in fuel cells.

Carbon aerogels are used in the construction of small electrochemical double layer supercapacitors. Due to the high surface area of the aerogel, these capacitors can be 2000 to 5000 times smaller than similarly rated electrolytic capacitors. Aerogel supercapacitors can have a very low impedance compared to normal supercapacitors and can absorb/produce very high peak currents.

### Aerogel composition:

The aerogel may be composed of any material used in prior art or any combination thereof.

The aerogel may be prepared using various oxide units in order to add properties required as additional to the storage and release of proteins. The invention includes silicon/titania; silicon/borate-; silicon/zirkonate but is not limited to these.

The aerogel may be adjusted by its composition to have hydrophilic or hydrophobic properties by use of a combination of silicate and dialkyl silicate units. The mixture can be in the range 100:0 to 80:20 such as such as 98:2, for example 96:4, such as 95:5, for example 94:6, such as 92:8, for example 90:10, such as 88:12, for example 86:14, such as 85:15, for example 84:16, such as 82:18. In one preferred embodiment the mixture can be in the range 95:5.

### Production of aerogels:

Silica aerogel is made by drying a hydrogel composed of colloidal silica in an extreme environment. Specifically, the process starts with a liquid alcohol like ethanol which is mixed with a silicon alkoxide precursor to form a silicon dioxide sol gel (silica gel).

Then, through a process called supercritical drying, the alcohol is removed from the gel. This is typically done by exchanging the ethanol for liquid acetone, allowing a better miscibility gradient, and then onto liquid carbon dioxide and then bringing the carbon dioxide above its critical point. A variant on this process involves the direct injection of supercritical carbon dioxide into the pressure vessel containing the aerogel.

The end result removes all liquid from the gel and replaces it with gas, without allowing the gel structure to collapse or lose volume.

Aerogel composites have been made using a variety of continuous and discontinuous reinforcements. The high aspect ratio of fibers such as fiberglass have been used to reinforce aerogel composites with significantly improved mechanical properties.

Resorcinol-formaldehyde aerogel (RF aerogel) is made in a way similar to production of silica aerogel.

Carbon aerogel is made from a resorcinol-formaldehyde aerogel by its pyrolysis in inert gas atmosphere, leaving a matrix of carbon. It is commercially available as solid shapes, powders, or composite paper.

### Physical and chemical properties of aerogels:

In one preferred embodiment the density of the aerogel is in the range from 0.05 to 1.0 g/mol such as 0.05 g/mol to 0.45 g/mol, for example 0.05 g/mol to 0.4 g/mol, such as 0.05 g/mol to 0.35 g/mol, for example 0.05 g/mol to 0.3 g/mol, such as 0.05 g/mol to 0.35 g/mol, for example 0.05 g/mol to 0.3 g/mol, such as 0.05 g/mol to 0.25 g/mol, for example 0.05 g/mol to 0.2 g/mol, such as 0.05 g/mol to 0.15 g/mol, for example 0.05 g/mol to 0.1 g/mol, such as 0.05 g/mol to 0.5 g/mol, for example 0.1 g/mol to 0.5 g/mol, such as 0.15 g/mol to 0.5 g/mol, for example 0.2 g/mol to 0.5 g/mol, such as 0.25 g/mol to 0.5 g/mol, for example 0.3 g/mol to 0.5 g/mol, such as 0.35 g/mol to 0.5 g/mol, for example 0.4 g/mol to 0.5 g/mol, such as 0.45 g/mol to 0.5 g/mol, for example 0.05 g/mol to 1.0 g/mol, such as 0.05 g/mol to 0.95 g/mol, for example 0.05 g/mol to 0.9 g/mol, such as 0.05 g/mol to 0.85 g/mol, for example 0.05 g/mol to 0.8 g/mol, such as 0.05 g/mol to 0.75 g/mol, for example 0.05 g/mol to 0.7 g/mol, such as 0.05 g/mol to 0.65 g/mol, for example 0.05 g/mol to 0.6 g/mol, such as 0.05 g/mol to 0.55 g/mol, for example 0.05 g/mol to 0.5 g/mol, such as 0.05 g/mol to 0.45 g/mol, for example 0.05 g/mol to 0.4 g/mol, such as 0.05 g/mol to 0.35 g/mol, for example 0.05 g/mol to 0.3 g/mol, such as 0.05 g/mol to 0.25 g/mol, for example 0.05 g/mol to 0.2 g/mol, such as 0.05 g/mol to 0.15 g/mol, for example 0.05 g/mol to 0.1 g/mol, such as 0.1 g/mol to 1.0 g/mol, for example 0.15 g/mol to 1.0 g/mol, such as 0.2 g/mol to 1.0 g/mol, for example 0.25 g/mol to 1.0 g/mol, such as 0.3 g/mol to 1.0 g/mol, for example 0.35 g/mol to 1.0 g/mol, such as 0.4 g/mol to 1.0 g/mol, for example 0.45 g/mol to 1.0 g/mol, such as 0.5 g/mol to 1.0 g/mol, for example 0.55 g/mol to 1.0 g/mol, such as 0.6 g/mol to 1.0 g/mol, for example 0.65 g/mol to 1.0 g/mol, such as 0.7 g/mol to 1.0 g/mol, for example 0.75 g/mol to 1.0 g/mol, such as 0.8 g/mol to 1.0 g/mol, for example 0.85 g/mol to 1.0 g/mol, such as 0.9 g/mol to 1.0 g/mol, for example 0.95 g/mol to 1.0 g/mol, such as 0.15 g/mol to 0.3 g/mol, for example 0.16 g/mol to 0.3 g/mol, such as 0.17 g/mol to 0.3 g/mol, for example 0.18 g/mol to 0.3 g/mol, such as 0.19 g/mol to 0.3 g/mol, for example 0.20 g/mol to 0.3 g/mol, such as 0.21 g/mol to 0.3 g/mol, for example 0.22g/mol to 0.3 g/mol, such as 0.23 g/mol to 0.3 g/mol, for example 0.24 g/mol to 0.3 g/mol, such as 0.25 g/mol to 0.3 g/mol, for example 0.26 g/mol to 0.3 g/mol, such as 0.27 g/mol to 0.3 g/mol, for example 0.28 g/mol to 0.3 g/mol, such as 0.29 g/mol to 0.3 g/mol, such as 0.15 g/mol to 0.29 g/mol, such as 0.15 g/mol to 0.28 g/mol, such as 0.15 g/mol to 0.27 g/mol, such as 0.15 g/mol to 0.26 g/mol, such as 0.15 g/mol to 0.25 g/mol, such as 0.15 g/mol to 0.24 g/mol, such as 0.15 g/mol to 0.23 g/mol, such as 0.15 g/mol to 0.22 g/mol, such as 0.15 g/mol to 0.21 g/mol, such as 0.15 g/mol to 0.20 g/mol, such as 0.15 g/mol to 0.19 g/mol, such as 0.15 g/mol to 0.18 g/mol, such as 0.15 g/mol to 0.17 g/mol, such as 0.15 g/mol to 0.16 g/mol.

In one embodiment the aerogel typically has a density of 0.15 g/mol to 0.30 g/mol.

The surface area of the aerogel is in one preferred embodiment in the range from 500 m²/g to 2000 m²/g such as from 500 m²/g to 1950 m²/g, for example from 500 m²/g to 1900 m²/g, such as from 500 m²/g to 1850 m²/g, for example from 500 m²/g to 1800 m²/g, such as from 500 m²/g to 1750 m²/g, for example from 500 m²/g to 1700 m²/g, such as from 500 m²/g to 1650 m²/g, for example from 500 m²/g to 1600 m²/g, such as from 500 m²/g to 1550 m²/g, for example from 500 m²/g to 1500 m²/g, such as from 500 m²/g to 1450 m²/g, for example from 500 m²/g to 1400 m²/g, such as from 500 m²/g to 1350 m²/g, for example from 500 m²/g to 1300 m²/g, such as from 500 m²/g to 1250 m²/g, for example from 500 m²/g to 1200 m²/g, such as from 500 m²/g to 1150 m²/g, for example from 500 m²/g to 1100 m²/g, such as from 500 m²/g to 1050 m²/g, for example from 500 m²/g to 1000 m²/g, such as from 500 m²/g to 950 m²/g, for example from 500 m²/g to 900 m²/g, such as from 500 m²/g to 850 m²/g, for example from 500 m²/g to 800 m²/g, such as from 500 m²/g to 750 m²/g, for example from 500 m²/g to 700 m²/g, such as from 500 m²/g to 650 m²/g, for example from 500 m²/g to 600 m²/g, such as from 500 m²/g to 550 m²/g, for example from 500 m²/g to 2000 m²/g, such as from 550 m²/g to 2000 m²/g, for example from 600 m²/g to 2000 m²/g, such as from 650 m²/g to 2000 m²/g, for example from 700 m²/g to 2000 m²/g, such as from 750 m²/g to 2000 m²/g, for example from 800 m²/g to 2000 m²/g, such as from 850 m²/g to 2000 m²/g, for example from 900 m²/g to 2000 m²/g, such as from 950 m²/g to 2000 m²/g, for example from 1000 m²/g to 2000 m²/g, such as from 1050 m²/g to 2000 m²/g, for example from 1100 m²/g to 2000 m²/g, such as from 1150 m²/g to 2000 m²/g, for example from 1200 m²/g to 2000 m²/g, such as from 1250 m²/g to 2000 m²/g, for example from 1300 m²/g to 2000 m²/g, such as from 1350 m²/g to 2000 m²/g, for example from 1400 m²/g to 2000 m²/g, such as from 1450 m²/g to 2000 m²/g, for example from 1500 m²/g to 2000 m²/g, such as from 1550 m²/g to 2000 m²/g, for example from 1600 m²/g to 2000 m²/g, such as from 1650 m²/g to 2000 m²/g, for example from 1700 m²/g to 2000 m²/g, such as from 1750 m²/g to 2000 m²/g, for example from 1800 m²/g to 2000 m²/g, such as from 1850 m²/g to 2000 m²/g, for example from 1900 m²/g to 2000 m²/g, such as from 1950 m²/g to 2000 m²/g, for example from 800 m²/g to 1500 m²/g, such as from 850 m²/g to 1500 m²/g, for example from 900 m²/g to 1500 m²/g, such as from 950 m²/g to 1500 m²/g, for example from 1000 m²/g to 1500 m²/g, such as from 1050 m²/g to 1500 m²/g, for example from 1100 m²/g to 1500 m²/g, such as from 1150 m²/g to 1500 m²/g, for example from 1200 m²lg to 1500 m²/g, such as from 1250 m²/g to 1500 m²/g, for example from 1300 m²/g to 1500 m²/g, such as from 1350 m²/g to 1500 m²/g, for example from 1400 m²/g to 1500 m²/g, such as from 1450 m²/g to 1500 m²/g, for example from 800 m²/g to 1450 m²/g, such as from 800 m²/g to 1400 m²/g, for example from 800 m²/g to 1350 m²/g, such as from 800 m²/g to 1300 m²/g, for example from 800 m²/g to 1250 m²/g, such as from 800 m²/g to 1200 m²/g, for example from 800 m²/g to 1150 m²/g, such as from 800 m²/g to 1100 m²/g, for example from 800 m²/g to 1050 m²/g, such as from 800 m²/g to 1000 m²/g, for example from 800 m²/g to 950 m²/g, such as from 800 m²/g to 900 m²/g, for example from 800 m²/g to 850 m²/g.

The surface area of the aerogel is in one preferred embodiment in the range from 800 to 1500 m2/g.

In one preferred embodiment the pore size of the aerogel is in the range of from 1 to 25 nm such as from 1 to 24 nm, for example from 1 to 22 nm, such as from 1 to 20 nm, for example from 1 to 18 nm, such as from 1 to 16 nm, for example from 1 to 14 nm, such as from 1 to 12 nm, for example from 1 to 10 nm, such as from 1 to 8 nm, for example from 1 to 6 nm, such as from 1 to 4 nm, for example from 1 to 2 nm, such as from 2 to 25 nm, for example from 4 to 25 nm, such as from 6 to 25 nm, for example from 8 to 25 nm, such as from 10 to 25 nm, for example from 12 to 25 nm, such as from 14 to 25 nm, for example from 16 to 25 nm, such as from 18 to 25 nm, for example from 20 to 25 nm, such as from 22 to 25 nm, for example from 24 to 25 nm, such as from 1 to 5 nm, for example from 5 to 10 nm, such as from 10 to 15 nm, for example from 15 to 20 nm, such as from 20 to 25 nm, for example from 2 to 10 nm, such as from 2 to 9 nm, for example from 2 to 8 nm, such as from 2 to 7 nm, for example from 2 to 6 nm, such as from 2 to 5 nm, for example from 2 to 4 nm, such as from 2 to 3 nm, for example from 3 to 10 nm, such as from 3 to 9 nm, for example from 3 to 8 nm, such as from 3 to 7 nm, for example from 3 to 6 nm, such as from 3 to 5 nm, for example from 3 to 4 nm, such as from 4 to 10 nm, for example from 4 to 9 nm, such as from 4 to 8 nm, for example from 4 to 7 nm, such as from 4 to 6 nm, for example from 4 to 5 nm, such as from 5 to 9 nm, for example from 5 to 8 nm, such as from 5 to 7 nm, for example from 5 to 6 nm, such as from 6 to 10 nm, for example from 6 to 9 nm, such as from 6 to 8 nm, for example from 6 to 7 nm, such as from 7 to 10 nm, for example from 7 to 9 nm, such as from 7 to 8 nm, for example from 8 to 10 nm, such as from 8 to 9 nm, for example from 9 to 10 nm.

The pore size of the aerogel is typically 2-5 nm.

The surface of the aerogels feel like a light but rigid foam, something between Styrofoam and the green floral foam used for arranging flowers. Aerogels are dry materials and do not resemble a gel in their physical properties but a nanofoam. Pressing softly on an aerogel typically does not leave a mark; pressing more firmly will leave a permanent dimple. Pressing firmly enough will cause a breakdown in the sparse structure, causing it to shatter like glass. Despite the fact that it is prone to shattering, it is very strong structurally. Its impressive load bearing abilities are due to the dendritic microstructure, in which spherical particles of average size 2-5 nm are fused together into clusters. These clusters form a three-dimensional highly porous structure of almost fractal chains, with pores smaller than 100 nm. The average size and density of the pores can be controlled during the manufacturing process.

Aerogels are remarkable thermal insulators because they almost nullify three methods of heat transfer (convection, conduction, and radiation). They are good convective inhibitors because air cannot circulate throughout the lattice. Silica aerogel is an especially good conductive insulator because silica is a poor conductor of heat-a metallic aerogel, on the other hand, would be a less effective insulator. Carbon aerogel is a good radiative insulator because carbon absorbs the infrared radiation that transfers heat.

Due to its hygroscopic nature, aerogel feels dry and acts as a strong desiccant.

Aerogels appear semi-transparent because they, in one preferred embodiment, consist of up to 99% air. The color it does have is due to Rayleight scattering of the shorter wavelengths of visible light by the nanosized dendritic structure. This causes it to appear bluish against dark backgrounds and whitish against bright backgrounds.

Aerogels by themselves are hydrophilic, but chemical treatment can make them hydrophobic. If they absorb moisture they usually suffer a structural change, such as contraction, and deteriorate, but degradation can be prevented by making them hydrophobic. Aerogels with hydrophobic interiors are less susceptible to degradation than aerogels with only an outer hydrophobic layer, even if a crack penetrates the surface. Hydrophobic treatment facilitates processing because it allows the use of awater jet cutter.

Aerogel can be used as drug delivery system due to its biocompatibility. Due to its high surface area and porous structure, drugs can be adsorbed from supercritical CO2. The release rate of the drugs can be tailored based on the properties of aerogel.

### Alteration of the surface properties of aerogels:

By means of functionalization, the surface properties of the aerogel according to the present invention can be altered; different functional groups can be used to change the properties of aerogel from hydrophilic to hydrophobic range. Controlling the surface coverage of the functional group as well as its type is one of the goals of this study. In principle, functionalization can be done by three basic methods:
1. Functionalization during the preparation of the gel (sol-gel).
2. Liquid phase functionalization (by placing the wet gel in functionalization solution).
3. Gas phase functionalization (by placing the aerogel in a gas stream of functionalization solution).

By optimizing the working conditions of each method, it is possible to control the functionalization degree of the desired functional group on the aerogel surface, and as a result the loading as well as the release time of the active drug can be tailored.

The hydrophobicity of the aerogel can in one preferred embodiment be adjusted by use of monomers containing alkyl groups on some silicium compositions.

### Bioactive agents encapsulated by aerogels:

One embodiment relates to encapsulation of one or more bioactive agents into an aerogel. The one or more bioactive agents can be, but is not limited to, the group consisting of proteins, peptides, enzymes, proteases, small organic or inorganic molecules, polysaccharides, pharmaceutical compositions or any combination thereof.

According to the present invention one or more enzyme(s) are encapsulated into one or more aerogel(s). In one embodiment encapsulation of one or more enzymes into one or more aerogels results in stability of the enzyme - such as unaltered activity over time. In another embodiment the activity of the enzyme is increased by encapsulation into the one or more aerogel(s).

In one embodiment one or more protease(s) are encapsulated in the aerogel. Proteases are in general difficult to store in solution because they digest each other. When proteases on the other hand are encapsulated in an aerogel they are not in contact with each other.

The present invention also relates to encapsulation of one or more subtilisins in the aerogel. Subtilisins comprises a family of serine proteases isolated from bacillus subtilis.

In another preferred embodiment the present invention relates to encapsulation of one or more hydrolytic enzymes. These hydrolytic enzymes comprises in one preferred embodiment hydrolytic enzymes that degrades polysaccharides and/or lipids.
In yet another embodiment the earogel encapsulates one or more oxidase(s). These oxidases can in one embodiment result in production of hydrogenperoxide. The present invention invention also relates to incorporation of a combination of starch and/or amylase and one or more oxidase(s) for generation of hydrogenperoxide.

In another embodiment the one or more enzymes are functionalized so it is actively incorporated into the three-dimensional structure of the aerogel. This leads to covalent attachment of the enzyme to the three-dimensional structure of the aerogel.

In one preferred embodiment a mixture of more than one enzyme is encapsulated into the aerogel. In another embodiment one or more enzymes and one or more other bioactive agent(s) are encapsulated into the same aerogel. In yet another embodiment more than one bioactive agent(s) are encapsulated into the same aerogel. In one preferred embodiment an enzyme and its substrate are encapsulated into the same aerogel.

In one preferred embodiment one or more bioactive agent(s) are encapsulated into an aerogel in a way so the one or more bioactive agent(s) are located in individual compartments in the aerogel.

In one embodiment the one or more enzymes can be selected from the group consisting of hemicellulolytically active enzymes, amylolytically active enzyme and/or cellulolytically active enzyme.

In another preferred embodiment the one or more bioactive agent(s) comprises endopeptidases.

In one embodiment the endopeptidase(s) comprises a Subtilisin (EC 3.4.21.62). The Subtilisin (EC 3.4.21.62) has the following characteristica: (i) optimum activity at a pH in the range of about 7 - 10, and (ii) optimum activity at a temperature in the range of about 55 - 65°C. The Subtilisin (EC 3.4.21.62) is in one embodiment Alcalase^{®}.

In one embodiment the hemicellulolytically active enzyme(s) is selected from the group consisting of Endo-1,4-beta-xylanase (E.C. 3.2.1.8), Xylan endo-1,3-beta-xylosidase (E.C. 3.2.1.32). Glucuronoarabinoxylan endo-1,4-beta-xylanase (E.C. 3.2.1.136), Betamannosidase (E.C. 3.2.1.25), Mannan endo-1,4-beta-mannosidase (E.C. 3.2.1.78) and Mannan endo-1,6-beta-mannosidase (E.C. 3.2.1.101). In another preferred embodiment the hemicellulolytically active enzyme is a xylanase. In one embodiment the xylanase is an endo-1,4-beta-xylanase (E.C. 3.2.1.8).

The amylolytically active enzyme(s) can in one preferred embodiment be an amylase.
In another embodiment the one or more amylolytically active enzyme(s) is selected from the group consisting of α- and β-amylases, amyloglucosidases (E.C. 3.2.1.3), pullulanases, α-1,6-endoglucanases, α-1,4-exoglucanases and isoamylases. The one or more amylolytically active enzyme(s) can also be amyloglucosidase. In one preferred embodiment the amyloglucosidase is an 1,4-alpha-glucosidase.

In one embodiment the anti-fouling composition agent comprises one or more aerogel(s) and at least one xylanase and at least one amyloglucosidase.

In another embodiment the anti-fouling composition agent comprises one or more aerogel(s) and at least one endo-1,4-beta-xylanase (E.C. 3.2.1.8) and at least one 1,4-alpha-glucosidase (E.C. 3.2.1.3).

In one embodiment the anti-fouling composition comprising one or more aerogel(s) and from about 0.1-10% of bioactive agent(s) by weight. In another preferred embodiment the anti-fouling composition comprising one or more aerogel(s) and from about 0.2-5% of bioactive agent(s) by weight. In yet another preferred embodiment the anti-fouling composition comprising one or more aerogel(s) and from about 0.5-1% of bioactive agent(s) by weight.

One or more bioactive agents can be encapsulated into the one or more aerogels. When two bioactive agents are encapsulated the present invention may relate in one embodiment to the combinations illustrated in table 1 and 2. The combinations not according to the present invention are designated with an c).

**Table 1: combination of bioactive agents for encapsulation into one or more aerogels.**

| | Proteins | Peptides | Enzymes | Proteases | Small organic molecules | Small inorganic molecules | Polysaccharides | Pharmaceutical compositions |
|---|---|---|---|---|---|---|---|---|
| Proteins | X^{c)} | X^{c)} | X | X | X^{c)} | X^{c)} | X^{c)} | X^{c)} |
| Peptides | x^{c)} | X^{c)} | X | X^{c)} | X^{c)} | X^{c)} | X^{c)} | X^{c)} |
| Enzymes | X | X | X | X | X | X | X | X |
| Proteases | X | X | X | X | X | X | X | X |
| Small organic molecules | X^{c)} | X^{c)} | X | X | X^{c)} | X^{c)} | X^{c)} | X^{c)} |
| Small inorganic molecules | X^{c)} | X^{c)} | X | X | X^{c)} | X^{c)} | X^{c)} | X^{c)} |
| Polysaccharides | X^{c)} | X^{c)} | X | X | X^{c)} | X^{c)} | X^{c}) | X^{c)} |
| Pharmaceutical compositions | X^{c)} | X^{c)} | X | X | X^{c)} | X^{c)} | X^{c)} | X^{c)} |

**Table 2: combination of bioactive agents for encapsulation into one or more aerogels.**

| | Oxidases | Endopeptidases | Proteases | Subtilisins | Hydrolytic enzymes | Hemicellulytically active enzymes | Amylolytically active enzymes | Cellulytically active enzymes |
|---|---|---|---|---|---|---|---|---|
| Oxidases | X | X | X | X | X | X | X | X |
| Endopeptidases | X | X | X | X | X | X | X | X |
| Protea-ses | X | X | X | X | X | X | X | X |
| Subtilisins | X | X | X | X | X | X | X | X |
| Hydrolytic enzymes | X | X | X | X | X | X | X | X |
| Hemicellulytically active enzymes | X | X | X | X | X | X | X | X |
| Amylolytically active enzymes | X | X | X | X | X | X | X | X |
| Cellulytically active enzymes | X | X | X | X | X | X | X | X |

In one embodiment two bioactive agents of the same type can be combined.

### Antifouling species generated by oxidase enzymes

The composition according to the present invention comprises at least one enzyme. In one embodiment the enzyme is an oxidase capable of acting on a compound, such as a substrate for said oxidase, wherein said action results in the formation of an antifouling species including an antimicrobial species comprising an antimicrobial activity, and wherein said compound does not form part of said coating composition.

In a more preferred embodiment the enzyme is an oxidase the activity of which results in the formation of a peroxide.

The oxidase can be present in said coating composition in combination with one or more additional enzymes including, but not limited to, an esterase, including a lipase, an amidase, including a protease, and a polysaccharide degrading enzyme, wherein said one or more additional enzyme(s), alone or in any combination, can be included in the presence or absence of one or more substrates for one or more of said enzymes.

The antifouling species comprising an antifouling activity is preferably generated when the at least one enzyme acts on a compound, or a precursor thereof including a polymer, capable of being secreted by a microbial organism. The compound can be a degradation product of a precursor compound, including a polymer secreted by and/or located on the surface of microbial organisms, wherein said degradation product is provided by a precursor enzyme acting on said precursor compound.

### Further antifouling species and enzymes resulting in their production

The species of the invention having antifoulant or antimicrobial activity can be any species capable of being produced e.g. as the result of an enzyme-substrate reaction. As such, there can be mentioned many species having antifouling activity, species having antibacterial/antifungal activity, species having biocidal activity, and species having biorepellent activity.

In one preferred embodiment the one or more aerogel(s) comprises one or more enzymes and one or more biocides.

The species having antimicrobial activity is thus produced by an enzymatic reaction between an enzyme and a substrate in the form of a compound which is preferably secreted by a microbial organism. Species having antimicrobial activity can be any species obtained as the direct result of enzymatic reaction between the enzyme and the compound, as well as any species formed from the product of such enzymatic reaction through further enzymatic and/or chemical reaction.

The compounds are not limited to microbial secretion products. The compounds of the invention can be any non-toxic compound supplied to a predetermined environment, such as a dock harbouring a ship hull, and capable of being converted into an antifouling species, including an antimicrobial species by the action of the at least one enzyme, including an oxidase.

Furthermore, it is also envisaged that antifouling species, including an antimicrobial species can be generated by a combination of i) enzymatic action on secreted microbial products, including polymers and degradation products thereof, and ii) enzymatic action on exogenously added compounds or precursor compounds, wherein said combination of enzymatic actions results in the formation of one or more antifouling species, including an antimicrobial species having an antimicrobial activity.

In accordance with the invention, the at least one enzyme, preferably an oxidase the activity of which results in the production of peroxide, including hydrogenperoxide, is comprised in the coating composition according to the invention in an effective amount to reduce or prevent fouling of a surface coated with the composition. In the present context the term "an effective amount" means an amount which is sufficient to control or eliminate or reduce or at least substantially reduce the settling of microbial organisms, plants and/or animals, including aquatic organisms such as bacteria, protozoa, algae and invertebrates, on a surface coated with the composition according to invention.

In order to test the amount of the at least one enzyme required in order to sufficiently reduce or prevent fouling, any type of standard or modified antifouling bioassay can be applied, including settlement assays as described by Willemsen (1994). In one presently preferred embodiment, the amount of the enzyme is in the range of from about 0.1 to preferably less than 10% (w/w) coating composition (dry weight), such as from about 0.1 to less than 9% (w/w), for example from about 0.1 to less than 8% (w/w), such as from about 0.1 to less than 7% (w/w), for example from about 0.1 to less than 6% (w/w), such as from about 0.1 to less than 5.5% (w/w), for example from about 0.1 to less than 5.0% (w/w), such as from about 0.1 to less than 4.5% (w/w), for example from about 0.1 to less than 4.0% (w/w), such as from about 0.1 to less than 3.5% (w/w), for example from about 0.1 to less than 3.0% (w/w), such as from about 0.1 to less than 2.5% (w/w), for example from about 0.1 to less than about 2.0% (w/w), such as from about 0.1 to less than about 1.5% (w/w), for example from about 0.1 to less than about 1.0% (w/w), such as from about 0.1 to less than about 0.5% (w/w).

In another embodiment the amount of the enzyme is present in the coating composition in the range of from about 0.2% (w/w) to about 0.4% (w/w) coating composition (dry weight), such as from about 0.4% (w/w) to about 0.6% (w/w), for example from about 0.6% (w/w) to about 0.8% (w/w) coating composition, such as from about 0.8% (w/w) to about 1.0% (w/w), for example from about 1.0% (w/w) to about 1.2% (w/w) coating composition, such as from about 1.2% (w/w) to about 1.4% (w/w), for example from about 1.4% (w/w) to about 1.6% (w/w) coating composition, such as from about 1.6% (w/w) to about 1.8% (w/w), for example from about 1.8% (w/w) to about 2.0% (w/w) coating composition, such as from about 2.0% (w/w) to about 2.5% (w/w), for example from about 2.5% (w/w) to about 3.0% (w/w) coating composition, such as from about 3.0% (w/w) to about 3.5% (w/w), for example from about 3.5% (w/w) to about 4.0% (w/w) coating composition, such as from about 4.0% (w/w) to about 4.5% (w/w), for example from about 4.5% (w/w) to about 5.0% (w/w) coating composition.

In a preferred embodiment the at least one enzyme is an oxidase the activity of which results in the formation of a peroxide, including hydrogen peroxide. The amount of hydrogen peroxide generated in accordance with the present invention depends on the amount of available compound on which the at least one oxidase can act. It will be possible to determine the amount of hydrogen peroxide generated by using the method of Janssen and Ruelius disclosed in Biochem. Biophys. Acta (1968), vol. 151, pages 330-342.

The amount of hydrogen peroxide generated is in preferred embodiments about or at least about 1 nmol/cm²/day, such as 2 nmol/cm²/day, for example 3 nmol/cm²/day, such as 4 nmol/cm²/day, for example 5 nmol/cm²/day, such as 2 nmol/cm²/day, for example 3 nmol/cm²/day, such as 4 nmol/cm²/day, for example 5 nmol/cm²/day, such as 6 nmol/cm²/day, for example 7 nmol/cm²/day, such as 8 nmol/cm²/day, for example 9 nmol/cm²/day, such as 10 nmol/cm²/day, for example 12 nmol/cm²/day, such as 14 nmol/cm²/day, for example 16 nmol/cm²/day, such as 18 nmol/cm²/day, for example 20 nmol/cm²/day, such as 22 nmol/cm²/day, for example 24 nmol/cm²/day, such as 26 nmol/cm²/day, for example 28 nmol/cm²/day, such as 30 nmol/cm²/day, for example 32 nmol/cm²/day, such as 34 nmol/cm²/day, for example 36 nmol/cm²/day, such as 38 nmol/cm²/day, for example 40 nmol/cm²/day, such as 42 nmol/cm²/day, for example 44 nmol/cm²/day, such as 46 nmol/cm²/day, for example 48 nmol/cm²/day, such as 50 nmol/cm²/day, for example 55 nmol/cm²/day, such as 60 nmol/cm²/day, for example 65 nmol/cm²/day, such as 70 nmol/cm²/day, for example 75 nmol/cm²/day, such as 80 nmol/cm²/day, for example 85 nmol/cm²/day, such as 90 nmol/cm²/day, for example 95 nmol/cm²/day, such as 100 nmol/cm²/day, for example 110 nmol/cm²/day, such as 120 nmol/cm²/day, for example 130 nmol/cm²/day, such as 140 nmol/cm²/day, for example 150 nmol/cm²/day, such as 160 nmol/cm²/day, for example 170 nmol/cm²/day, such as 180 nmol/cm²/day, for example 190 nmol/cm²/day, such as 200 nmol/cm²/day, for example 220 nmol/cm²/day, such as 240 nmol/cm²/day, for example 260 nmol/cm²/day, such as 280 nmol/cm²/day, for example 300 nmol/cm²/day, such as 320 nmol/cm²/day, for example 340 nmol/cm²/day, such as 360 nmol/cm²/day, for example 380 nmol/cm²/day, such as 400 nmol/cm²/day, for example 420 nmol/cm²/day, such as 440 nmol/cm²/day, for example 460 nmol/cm²/day, such as 480 nmol/cm²/day, for example 500 nmol/cm²/day, such as 520 nmol/cm²/day, for example 540 nmol/cm²/day, such as 560 nmol/cm²/day, for example 580 nmol/cm²/day, such as 600 nmol/cm²/day, for example 620 nmol/cm²/day, such as 640 nmol/cm²/day, for example 660 nmol/cm²/day, such as 680 nmol/cm²/day, for example 700 nmol/cm²/day, such as 720 nmol/cm²/day, for example 740 nmol/cm²/day, such as 760 nmol/cm²/day, for example 780 nmol/cm²/day, such as 800 nmol/cm²/day, for example 820 nmol/cm²/day, such as 840 nmol/cm²/day, for example 860 nmol/cm²/day, such as 880 nmol/cm²/day, for example 900 nmol/cm²/day, such as 920 nmol/cm²/day, for example 940 nmol/cm²/day, such as 960 nmol/cm²/day, for example 980 nmol/cm²/day, such as 1000 nmol/cm²/day.

Preferred oxidases include, but is not limited to, malate oxidase; glucose oxidase; hexose oxidase; cholesterol oxidase; arylalcohol oxidase: galactose oxidase; alcohol oxidase; lathosterol oxidase; aspartate oxidase; L-amino-acid oxidase; D-amino-acid oxidase; amine oxidase; D-glutamate oxidase; ethanolamine oxidase; NADH oxidase; urate oxidase (uricase); superoxide dismutase; and the like.

In one preferred embodiment the at least one enzyme is a hexose oxidase, including, but not limited to any oxidoreductase of class EC 1.1.3.5. Hexose oxidases are enzymes which in the presence of oxygen is capable of oxidising D-glucose and several other reducing sugars including maltose, lactose and cellobiose to their corresponding lactones with subsequent hydrolysis to the respective aldobionic acids. Hexose oxidase differs from another oxidoreductase, glucose oxidase, which can only convert D-glucose, in that the enzyme can utilise a broader range of sugar substrates.

Hexose oxidase is produced naturally by several marine algal species. Such species are found inter alia in the family Gigartinaceae. In one preferred embodiment the hexose oxidase is obtained from the marine algae Chondrus cripus. Reference is made to EP 0 832 245. WO 96/40935 and WO 98/13478 also disclose the cloning and expression in recombinant host organisms of a gene encoding a protein with HOX activity.

In another preferred embodiment the compound and the enzyme, respectively, is selected from glucose / hexose oxidase; glucose / glucose oxidase; L amino acid / L amino acid oxidase; galactose / galactose oxidase; lactose / beta-galactosidase / hexose oxidase; 2-deoxyglucose / glucose oxidase; pyranose / pyranose oxidase; and mixtures thereof.

### Precursor enzymes

The antifouling species, including an antimicrobial species can be generated directly by the action of the at least one enzyme, optionally in combination with an initial action of one or more precursor enzymes. In the latter case, the precursor enzyme(s) and the precursor compound(s) are selected such that the precursor enzyme(s) eventually generates the compound.

An example of a precursor enzyme is any polysaccharide digesting enzyme, including amyloglucosidase, and an example of a precursor compound is any polysaccharide.

Thus in one embodiment the coating composition can comprise at least one oxidase such as e.g. hexose oxidase and at least one amylolytically active enzyme, such as e.g. an amyloglucosidase, and/or at least one hemicellulolytically active enzyme, such as e.g. a xylanase, and/or at least one cellulolytically active enzyme, such as e.g. a cellulase, including any combination of an oxidase with the aforementioned polysaccharide degrading enzymes, such as an oxidase and an amylolytically active enzyme, an oxidase and a hemicellulolytically active enzyme, an oxidase and a cellulolytically active enzyme, an oxidase and an amylolytically active enzyme and a hemicellulolytically active enzyme, such as an oxidase and an amylolytically active enzyme and a cellulolytically active enzyme, and an oxidase and a hemicellulolytically active enzyme and an cellulolytically active enzyme.

A number of other enzymes, as alternatives to an oxidase, or in addition to oxidases, can be employed in accordance with the present invention, either alone or in any combination, including a combination wherein the at least one oxidase is also present.

### Esterases and lipases

Esterases and lipases are triacylglycerol hydrolysing enzymes capable of splitting of fatty acids having short, medium and long chain lengths. Esterases and lipases degrade cell wall lipids and other lipid associated macromolecules at the surface of microbial organisms.

Accordingly, in one embodiment the at least one enzyme is an esterase and the compound is an ester bond-containing species. Examples of esterases include, but is not limited to, carboxylesterase, arylesterase, acetylesterase, and the like.

In yet another embodiment the at least one enzyme/precursor enzyme is a lipase such as, but not limited to, triacylglycerol lipase, lipoprotein lipase, and the like.

### Proteases

Proteinaceous materials involved in fouling the surfaces are subject to disruption by proteases. Families of proteolytic enzymes are well known, as reviewed in Neurath, Science 224, 350-357, 1984. Candidates for use in non-toxic anti-fouling coating compositions can be drawn from these families, trypsin and subtilisn being an example of serine proteases of type I and II, papain being an example of a sulfhydryl protease, pepsin being an example of an acid protease, carboxypeptidase A and B and thermolysin being examples of metalloproteases of type I and II. Other protease families of relevance are the aminopeptidases, the collagenases and the calcium and ATP-activiated proteases, each with numerous examples.

Accordingly, in a still further embodiment the at least one enzyme/precursor enzyme is a protease such as, but not limited to, subtilisins, chymotrypsins, trypsins, elastases, cathepsins, papains, chromopapains, pepsins, carboxypeptidase A, carboxypeptidase B, thermolysins, calcium activated proteases, ATP-activated proteases, exopeptidases such as aminopeptidases and carboxypeptidases, endopeptidases, and the like.

One class of preferred enzymes are the subtilisins. Subtilisins are serine endopeptidases. Examples include subtilisin BPN' (also known as subtilisin B, subtilopeptidase B, subtilopeptidase C, Nagarse, Nagarse proteinase, subtilisin Novo, bacterial proteinase Novo) and subtilisin Carlsberg (subtilisin A, subtilopeptidase A, alcalase Novo). Now grouped under IUBMB enzyme nomenclature EC 3.4.21.62, formerly EC 3.4.4.16 and included in EC 3.4.21.14. Subtilisin enzymes are produced by various *Bacillus subtilis* strains and other *Bacillus* species.

Further examples of subtilisins include, but is not limited to, e.g. alcalase; alcalase 0.6L; alcalase 2.5L; ALK-enzyme; bacillopeptidase A; bacillopeptidase B; *Bacillus subtilis* alkaline proteinase bioprase; bioprase AL 15; bioprase APL 30; colistinase; (see also comments); subtilisin J; subtilisin S41; subtilisin Sendai; subtilisin GX; subtilisin E; subtilisin BL; genenase I; esperase; maxatase; alcalase; thermoase PC 10; protease XXVII; thermoase; superase; subtilisin DY; subtilopeptidase; SP 266; savinase 8.0L; savinase 4.0T; kazusase; protease VIII; opticlean; Bacillus subtilis alkaline proteinase; protin A 3L; savinase; savinase 16.0L; savinase 32.0 L EX; orientase 10B; protease S.

Accordingly, one particularly preferred protease is endopeptidases of the subtilisin type (EC 3.4.21.62). Subtilisin type proteases can be applied in the form of a commercially available enzyme preparations such as Alcalase^{®}. Alcalase^{®} is a serine-type protease characterised by a good performance at elevated temperatures and moderate alkalinity. In a presently preferred embodiment the enzyme preparation Alcalase 2.5 L, Type DX^{®} is applied. However it is also contemplated that other Alcalase^{®} products, including Alcalase 2.0 T^{®}, Alcalase 3.0 T^{®} and Alcalase 2.5 L, Type DX^{®}, can be applied in accordance with the present invention. Such Alcalase^{®} enzyme preparations are available from Novozymes (Novozymes, Novo Allé, 2880 Bagsvaerd, Denmark).

However, it is also within the scope of the invention that other proteases having essentially the same characteristics as the protease of Alcalase^{®} can be successfully applied in accordance with the invention. Thus, it is contemplated that other proteases, such as subtilisins, having essentially the same temperature and pH profiles as the Alcalase, can be utilised. The temperature and pH profiles of the Alcalase can be found on the product sheet from Novozyme A/S (B259f-GB).

Accordingly, it is within the scope of the invention that a subtilisin type protease (EC 3.4.21.62) having the following characteristica: (i) optimum activity at a pH in the range of about 7 to 10, such as from more than 7.5 to about 10; and (ii) optimum activity at a temperature in the range of from about or more than 55 to about 65°C, may advantageously be applied.

### Polysaccharide degrading enzymes

Enzymes/precursor enzymes capable of degrading polysaccharides are generally desirable in combination with an oxidase the activity of which results in the production of peroxide. The reason is that polysaccharide digesting enzymes can break down a polysaccharide component of a microbial adhesive structure and/or degrade important structural polysaccharides of microorganisms into building blocks of preferably mono- and/or disaccharides. Such compounds and precursors thereof are substrates for oxidases and their formation thus enhances the subsequent production of peroxides. Additionally, the polysaccharide digesting enzymes of the present invention can prevent or interfere with the attachment process or the subsequent growth, metamorphosis or replication of the fouling organisms in question.

Accordingly, in a still further embodiment the at least one enzyme/precursor enzyme is a polysaccharide digesting enzyme, such as, but not limited to, alpha-amylase, beta-amylase, beta-glucosidase, glucosidase, glycosidase, cellulase, pectinase, hyaluonidase, beta-glucuronidase.

The enzymes beta-amylase, beta-glucosidase, and glycosidase all belong to the group of enzymes that can degrade polysaccharides. Pectinase and cellulase are enzymes which break down pectin and cellulose, respectively, two ubiquitous structural polymers of the plant cell wall and cell wall connective tissue matrix. Lysozyme and achromopeptidase can also break cell walls, the latter having an exceptional range of activity against microorganisms. Hyaluronic acid and collagen have analogous structural roles in animals and are degraded by hyaluronidase and collagenase, respectively. Beta-Glucuronidase will also break down hyaluronic acid.

Additionally preferred polysaccharide degrading enzymes are "hemicellulolytically active" enzymes, "cellulolytically active" enzymes, and "amylolytically active" enzymes. The the first group belong enzymes such as xylanases, which have the capability to degrade at least one substance belonging to the group of compounds and precursor compounds generally referred to as hemicellulose, including xylans and mannans, such as Endo-1,4-beta-xylanase (E.C. 3.2.1.8), Xylan endo-1,3-beta-xylosidase (E.C. 3.2.1.32), Glucuronoarabinoxylan endo-1,4-beta-xylanase (E.C. 3.2.1.136), Betamannosidase (E.C. 3.2.1.25), Mannan endo-1,4-beta-mannosidase (E.C. 3.2.1.78) and Mannan endo-1,6-beta-mannosidase (E.C. 3.2.1.101).

Enzymes having "cellulolytic activity" are also generally referred to as cellulases and is used herein to designate any cellulose hydrolysing enzyme.

"Amylolytically active" enzymes includes, in the present context, amylases, such as α-amylases and β-amylases, amyloglucosidases, pullulanases, α-1,6-endoglucanases, α-1,4-exoglucanases and isoamylases.

The above-mentioned enzymes occur in preferred embodiments in combination with at least one oxidase. Accordingly, when the coating composition e.g. comprises an aerogel which comprises an oxidase capable of acting on a compound, wherein said action results in the formation of an antimicobial species, the aerogel and/or coating composition can in further embodiments comprise one or more of
at least one esterase from the above group, optionally in the absence of a substrate for said esterase, and/or
at least one lipase from the above group, optionally in the absence of a substrate for said lipase, and/or
at least one protease from the above group, optionally in the absence of a substrate for said protease, and/or
at least one polysaccharide degrading enzyme from the above group, optionally in the absence of a substrate for said enzyme.

Preferred combinations of the above enzymes in combination with the at least one oxidase include
a coating composition comprising at least one oxidase in the absence of a substrate for said oxidase and at least one hydrolytic enzyme, optionally in the absence of a substrate for such a hydrolytic enzyme,
a coating composition comprising at least one oxidase in the absence of a substrate for said oxidase and at least one esterase,
a coating composition comprising at least one oxidase in the absence of a substrate for said oxidase and at least one lipase,
a coating composition comprising at least one oxidase in the absence of a substrate for said oxidase and at least one protease,
a coating composition comprising at least one oxidase in the absence of a substrate for said oxidase and at least one polysaccharide digesting enzyme,
a coating composition comprising at least one oxidase in the absence of a substrate for said oxidase and at least one esterase and at least one lipase,
a coating composition comprising at least one oxidase in the absence of a substrate for said oxidase and at least one esterase and at least one protease,
a coating composition comprising at least one oxidase in the absence of a substrate for said oxidase and at least one esterase and at least one polysaccharide digesting enzyme,
a coating composition comprising at least one oxidase in the absence of a substrate for said oxidase and at least one esterase and at least one lipase and at least one protease,
a coating composition comprising at least one oxidase in the absence of a substrate for said oxidase and at least one esterase and at least one lipase and at least one polysaccharide digesting enzyme,
a coating composition comprising at least one oxidase in the absence of a substrate for said oxidase and at least one esterase and at least one lipase and at least one protease and at least one polysaccharide digesting enzyme,
a coating composition comprising at least one oxidase in the absence of a substrate for said oxidase and at least one lipase and at least one protease,
a coating composition comprising at least one oxidase in the absence of a substrate for said oxidase and at least one lipase and at least one protease and at least one polysaccharide digesting enzyme, and
a coating composition comprising at least one oxidase in the absence of a substrate for said oxidase and at least one protease and at least one polysaccharide digesting enzyme.

Accordingly, it will be understood that the substrate for the one or more enzymes can be present or not present in the coating composition and/or the aerogel.

In various embodiments, the above coating compositions comprising an aerogel do not comprise a substrate for the one or more enzyme(s) and/or the one or more precursor enzyme(s) employed. Accordingly, there are provided embodiments wherein any one of the above-mentioned coating compositions i) does not comprise any substrate for the at least one esterase, when an esterase is present, ii) does not comprise any substrate for the at least one lipase, when a lipase is present, iii) does not comprise any substrate for the at least one protease, when a protease is present, and iv) does not comprise any substrate for the at least one polysaccharide digesting enzyme, when a polysaccharide digesting enzyme is present, v) does not comprise the substrate for the precursor enzyme.

In further embodiments the above coating compositions according to the invention i) do not comprise a substrate for an esterase and a lipase, when at least an esterase and a lipase are present, optionally in combination with further enzymes ii) do not comprise a substrate for an esterase and a protease, when at least an esterase and a protease are present, optionally in combination with further enzymes, iii) do not comprise a substrate for an esterase and a polysaccharide digesting enzyme, when at least an esterase and a polysaccharide digesting enzyme are present, optionally in combination with further enzymes, iv) do not comprise a substrate for an lipase and a protease, when at least a lipase and a protease are present, optionally in combination with further enzymes, v) do not comprise a substrate for a lipase and a polysaccharide digesting enzyme, when at least a lipase and a polysaccharide digesting enzyme are present, optionally in combination with further enzymes, and vi) do not comprise a substrate for a protease and a polysaccharide digesting enzyme, when at least a protease and a polysaccharide digesting enzyme are present, optionally in combination with further enzymes.

In the present invention, the at least one enzyme comprised in the coating composition can be any one or more of a purified enzyme or a crude enzyme. The source of the enzyme includes microorganisms, plants, and animals. When incorporating an enzyme into the coating composition, the enzyme may be directly incorporated or it can be used after modification with another species, or in the form of an immobilized enzyme. Immobilization includes enzymes entrapped in reverse micelles; enzymes modified with lipids or surfactants; enzymes modified with polyethylene glycol; and enzymes immobilized on polymer matrices, among other forms.

### Rosins

It is in one embodiment preferred to include into a coating composition of the invention at least one rosin. Rosins are solid materials that e.g. occur naturally in the oleo rosin of pine trees and is typically derived from the oleo resinous exudate of the living tree, from aged stumps and from tall oil produced as a by-product of kraft paper manufacture.

Rosin compounds have a number of highly desirable properties for use as binders in antifouling paints such as e.g. being fairly non-toxic to humans, being compatible with a large number of other binders and being relatively inexpensive and readily available from natural resources.

Thus, rosins are used in paints as binders, and thereby provide a rather non-toxic alternative to synthetic and more toxic binders such as e.g. polymeric binder components as epoxy, polyvinylacetate, polyvinylbutyrate and polyvinylchloride acetate.

Rosin is typically classed as gum rosin, wood rosin, or as tall oil rosin which indicates its source. The rosin materials can be used unmodified, in the form of esters of polyhydric alcohols, in the form of rosins polymerised through the inherent unsaturation of the molecules or in the form of hydrogenated rosin. Thus, rosin can be further treated by e.g. hydrogenation, dehydrogenation, polymerisation, esterification, and other post treatment processes. Additionally, rosin with e.g. free carboxylic acid groups are capable of reacting with metals and thereby forming rosin metal salts.

Accordingly, the rosin compound of the antifouling paint composition of the present invention is at least one selected from rosins, rosin derivatives, and rosin metal salts. Examples of rosins include tall rosin, gum rosin, and wood rosin. Examples of rosin derivatives include hydrogenated rosins, modified rosins obtained by reacting rosins with maleic anhydride, formylated rosins, and polymerised rosins. Examples of rosin metal salts include zinc rosinates, calcium rosinates, copper rosinates, magnesium rosinates, and products of the reaction of rosins with compounds of other metals.

Rosins of natural origin have the beneficial effect that when used in combination with enzymes, the activity of said enzymes are not substantially affected by the rosins as compared to enzymes in paint compositions prepared with synthetic binders of non-natural origin. Accordingly, it was found that no enzyme activity was present in paint compositions comprising protease and synthetic binders of non-natural origin.

The rosins are furthermore believed to have an immobilising effect on the enzymes and thus preventing the enzymes from being released from the paint composition into the environment.

The composition according to invention comprises a rosin compound wherein the content of the rosin compound is in the range of from about 5 to about 60% by weight. It is preferred that the amount of rosin compound is higher than about 10% such as up to about 20% by weight. However, it is also contemplated that the amount of rosin compound in the composition can be up to about 30%, such as up to about 40%, up to about 50% and up to about 55%. Thus, a pigmented composition according to the invention could advantageously comprise an amount of rosin compound in the range of about 10-30% by weight, and a lacquer composition could comprise up to about 60% of rosin compound by weight.

### Resins

As an alternative to rosin compounds, any suitable resin compound can be employed, such as the resins described below.

The resin produced by most plants is a viscous liquid, typically composed mainly of volatile fluid terpenes, with lesser components of dissolved non-volatile solids which make resin thick and sticky. The most common terpenes in resin are the bicyclic terpenes alpha-pinene, beta-pinene, delta-3 carene and sabinene, the monocyclic terpenes limonene and terpinolene, and smaller amounts of the tricyclic sesquiterpenes longifolene, caryophyllene and delta-cadinene. Some resins also contain a high proportion of resin acids. The individual components of resin can be separated by fractional distillation

A few plants produce resins with different compositions, most notably Jeffrey Pine and Gray Pine, the volatile components of which are largely pure n-heptane with little or no terpenes. The exceptional purity of the n-heptane distilled from Jeffrey Pine resin, unmixed with other isomers of heptane, led to its being used as the defining zero point on the octane rating scale of petrol quality. Because heptane is highly flammable, distillation of resins containing it is very dangerous. Some resin distilleries in California exploded because they mistook Jeffrey Pine for the similar but terpene-producing Ponderosa Pine.

Some resins when soft are known as *oleo-resins,* and when containing benzoic acid or cinnamic acid they are called balsams. Other resinous products in their natural condition are a mix with gum or mucilaginous substances and known as gum resins. Many compound resins have distinct and characteristic odors, from their admixture with essential oils.

Certain resins are obtained in a fossilized condition, amber being the most notable instance of this class; African copal and the kauri gum of New Zealand are also procured in a semi-fossil condition.

Solidified resin from which the volatile terpene components have been removed by distillation is known as rosin. Typical rosin is a transparent or translucent mass, with a vitreous fracture and a faintly yellow or brown colour, non-odorous or having only a slight turpentine odour and taste.
1) It is insoluble in water, mostly soluble in alcohol, essential oils, ether and hot fatty oils, 2)softens and melts under the influence of heat, is not capable of sublimation, and burns with a bright but smoky flame.

This comprises a complex mixture of different substances including organic acids named the resin acids. These are closely related to the terpenes, and derive from them through partial oxidation. Resin acids can be dissolved in alkalis to form resin soaps, from which the purified resin acids are regenerated by treatment with acids. Examples of resin acids are abietic acid (sylvic acid), C₂₀H₃₀O₂, plicatic acid contained in cedar, and pimaric acid, C₂₀H₃₅O₂, a constituent of gallipot resin. Abietic acid can also be extracted from rosin by means of hot alcohol; it crystallizes in leaflets, and on oxidation yields trimellitic acid, isophthalic acid and terebic acid. Pimaric acid closely resembles abietic acid into which it passes when distilled in a vacuum; it has been supposed to consist of three isomers.

Synthetic resins are materials with similar properties to natural resins-viscous liquids capable of hardening. They are typically manufactured by esterification or soaping of organic compounds. The classic variety is epoxy resin, manufactured through polymerization-polyaddition or polycondensation reactions, used as a thermoset polymer for adhesives and composites. One more category, which constitutes 75% of resins used, is unsaturated polyester resin. Ion exchange resin is another important class with application in water purification and catalysis of organic reactions. Other examples of resin includes AT-10 Resin and melamine resin.

### Repellants

In addition to the at least one enzyme capable of producing an antifouling species, including an antimicrobial species and means for immobilization thereof, including rosins, as described above, the coating composition of the invention can also comprise additional agents useful for preventing fouling, particularly macrofouling. One such group of agents is termed repellants of the macrofouling organisms. Repellants belong to a group of biologically active compounds which repell rather than attract microbial organisms.

Repellants according to the invention include molecules that are customarily associated with some inimicable material formed by a predator (or other non-compatible organism) of the macrofouling organism. An example is the material customarily excreted by starfish that causes such prey organism as scallops to immediately react to the material and try to escape therefrom. When affixed to a surface as described herein, the repellant would not freely diffuse but would act to elicit the escape response when the organism contacted the surface being protected. An example of this would be a purified chemical repellant or an impure suspension containing the active chemical repellant that is obtained by grinding and partially fractionating a coral or algae preparation. The repellants of choice are those natural products used by corals, seaweeds and other aquatic organisms to avoid fouling of their surfaces.

### Surfactants

In addition to natural products that can act as repellants, the surface protection can also be brought about by affixing a surfactant. Some repellants will be surfactants and vice versa, but as surfactants are generally not regarded as repellants in all senses of the word, they are considered as a separate class of bioactive agents having a useful effect in combination with enzymes and/or repellants of this invention.

A surfactant can have an inhibitory effect on attachment of organisms to a surface even when immobilized on or within a coating composition of the invention. Specific examples of immobilized surfactants are cationic, anionic and non-ionic surfactants such as quaternary ammonium ions, dipalmitoyl phosphatidyl choline, aralkyl sulfonates and sucrose esters, respectively. Other examples are set forth in the Kirk-Othmer Encyclopedia of Chemical Technology, Vol. 22, pages 332-432, John Wiley & Sons, New York, 1983.

### Tannic acids

Yet another example of a compound capable of being incorporated into coating compositions according to the invention is tannic acid, a representative compound of the tannins, a family of compounds secreted by certain species of marine brown algae (e.g. Sargassum), which appear to restrict bacterial colonization of the frond surface (Sieburth and Conover (1965) Nature 208 52). This is exemplary of the class of compounds, useful in non-toxic anti-fouling coatings, that act by interference with enzymatic reactions necessary for attachment of macro- or micro-organisms. Candidate compounds in this category include kojic acid and similar inhibitors of polyphenol oxidase. These inhibitors will interfere with the cross-linking of cement-forming materials, of similar value are glucosyl transferase inhibitors which will prevent the formation of polysaccharide adhesives used in adhesion, mutastein, ribocitrin, 1-deoxynojirimycin, acarbose, and N-methyldeoxynojirimycin being exemplary of these.

### Function of encapsulation

In one preferred embodiment one or more bioactive agent(s) are encapsulated into an aerogel to stabilize the one or more bioactive agent(s).

In one preferred embodiment one or more bioactive agent(s) are encapsulated into an aerogel to retain or improve the activity of the one or more bioactive agent(s).

In one preferred embodiment one or more bioactive agent(s) are encapsulated into an aerogel to retain or improve the heat stability of the one or more bioactive agent(s).

### Degradation of aerogels

Aerogels are degraded over time. Aerogels comprises a self-polishing effect.
The one or more encapsulated bioactive agents will be exposed to the surface over time. In one embodiment the one or more encapsulated bioactive agents will be released from the one or more aerogel(s) by controlled release.

In one embodiment hydrolysis of the hydrolysable moieties of the coating composition generates a self-polishing effect. Being submerged in water the hydrolysable moieties will slowly hydrolyse at the interface between the coating composition and the water phase. When sufficient hydrophilic groups have been formed, the coating composition becomes water-soluble and dissolves leading to a "self-polishing" effect.

In one preferred embodiment the self-polishing effect comprises a true self-polishing effect. In another embodiment the self-polishing effect comprises a simple self-polishing effect

The leaching of the one or more encapsulated bioactive agent(s) is slow or absent when the aerogel is exposed to water or another liquid over periods of hours. The leaching will be 0 to 2% per hour at 37°C calculated based on total encapsulated protein.

The leaching of the one or more encapsulated bioactive agent(s) from the aerogel can be modified by alteration of the aerogel composition and/or by annealing.

### Use of aerogels for anti-fouling:

In one embodiment the fouling organisms comprises aquatic organisms selected from the group consisting of bacteria, protozoa, fungus, algae and invertebrates. In one preferred embodiment the aquatic organism is selected from barnacles and mussels. In another embodiment the aquatic organism are of the *Cirripedia* subclass including *Balanus galeatus, Balanus amphitrite, Elminius modestus, Balanus improvisus* and *Balanus balanoides.*

In one preferred embodiment the aerogel which comprises one or more encapsulated bioactive agents has an anti-fouling effect and or anti-epibiosis effect. Anti-fouling is the process of removing or inhibiting the accumulation of biofouling.

Biofouling or biological fouling is the undesirable accumulation of microorganisms, plants, algae, and animals on surfaces such as submerged structures like ships' hulls. Biofouling also occurs on the surfaces of living marine organisms, when it is known as epibiosis. Biofouling is also found in membrane systems, such as membrane bioreactors and reverse osmosis spiral wound membranes. In the same manner it is found as fouling in cooling water cycles of large industrial equipments and power stations.

Biofouling is divided into microfouling - biofilm formation and bacterial adhesion - and macrofouling - attachment of larger organisms, of which the main culprits are barnacles, mussels, polychaete worms, bryozoans, and seaweed. Together, these organisms form a fouling community.

Individually small, accumulated biofoulers can form enormous masses that severely diminish ships' maneuverability and carrying capacity. Fouling causes huge material and economic costs in maintenance of marineculture, shipping industries, naval vessels, and seawater pipelines.

Biofouling can occur on any surface submerged in water such as for example on ships. Other examples of surfaces that can be exposed to biofouling are any installations, membranes, nets, measuring equipment or other equipment in aquaculture.

Biofouling can also occur in groundwater wells where buildup can limit recovery flow rates, and in the exterior and interior of ocean-laying pipes. In the latter case it has been shown to retard the seawater flow through the pipe and has to be removed with the tube cleaning process.

In one preferred embodiment the surface for application of the anti-fouling composition is a surface that is at least occasionally immersed in water, wherein said water includes fresh, salt or brackish water. The surface can be selected from the group consisting of the surfaces of vessels including boats and ships, ship hulls, off-shore equipment, pipes, substructures of bridges, piers and aquacultural apparatuses including fish farming nets.

In another preferred embodiment the aerogel which comprises one or more encapsulated bioactive agents has an anti-bacterial effect. The aerogel with the anti-bacterial effect can in one preferred embodiment be employed in food production such as in the dairy industry. In another embodiment the aerogel with the anti-bacterial effect can be used in hospitals such as in an operating room.

### Antimicrobial effects of the coating composition

The coating compositions of the invention are capable of reducing and/or eliminating fouling in the form of microbial growth and/or the formation of bio-film on objects coated with the composition. The microbial organisms can be e.g. bacteria, vira, fungal cells and slime molds. For aquatic environments, the microbial organisms are marine organisms.

In selecting the at least one enzyme of the coating composition one must take into consideration - among other things - the type of surface being protected, the environment in which the surface is found, and the organism against which protection is being sought.

The general principle underlying the choice of enzyme to be immobilized is that the abundance of a particular type of enzyme should be proportional to the probable frequency of surface contact with the target organism against which the antifouling species, including an antimicrobial species generated by the enzyme has anti-fouling efficacy.

### Marine antifouling effects

As an example, a short-term protection against settling organisms in a marine environment can focus on deterring the formation of films that are deposited by the settlement and growth of marine algae and bacteria. In this case, the bioactive materials to be incorporated on the surface can be distributed equally between a bactericide and an algaecide.

Accordingly, the antimicrobial effects of the compositions according to the invention are directed to - among others - the following groups of microbial organisms: Bacteria, fungi, algae, protozoa, porifera, coelenterata, platyhelminthes, nemertea, rotifera, bryozoa, brachiopoda, annelida, arthropoda, mollusca, echinodermata and chordata.

One interesting case is that of preventing growth and/or attachment to a surface of Vibrio species in an aquatic environment. Vibrio species often cluster together due to the presence of an extracellular polysaccharide (slime) that they synthesize. The best-known species of *Vibrio* is *V. cholerae* which causes cholera, a severe diarrhoeal disease resulting from a toxin produced by bacterial growth in the gut. Accordingly, the present invention in one preferred embodiment also relates to preventing and /or reducing the risk of cholera outbreaks in environments wherein *V. cholerae* is present. The method includes the step of coating pipes, filters, tanks and the like with a composition according to the invention comprising at least one oxidase and a polysaccharide degrading enzyme capable of degrading polysaccharides secreted by Vibrio species including *V. cholerae.*

The development of an antifouling species, including an antimicrobial species which could eliminate only, for example, barnacles in an aqueous environment would be solving only part of the fouling problem. Studies on the temporal development of a fouling community have revealed that bacteria are usually the first organisms to colonize a submerged surface. Attached bacteria produce a secondary extracellular polymeric adhesive, and eventually the surface of the substratum becomes coated with bacteria embedded within this extracellular matrix (collectively referred to as a bacterial film).

The rate of subsequent colonization by other microorganisms, and by marine invertebrate larvae, is often dependent upon the initial formation of a bacterial film. Consequently, the development of a coating composition capable of reducing and/or eliminating the process of bacterial film formation can be expected also to have a significant anti-fouling effect.

A small number of proteins and carbohydrates constitute the important structural elements of the cell wall of a wide range of microbial organisms. Collagen, cellulose, and chitin are three abundent structural polymers. Chitin, for example, is an important constitutent of the shell matrix of the inarticulate Brachipoda, the exoskeleton of the Ectoprocta (e.g. Bryozoa), the walls of sponge gemmules (the dispersal stage of the sponge life cycle), the perisarc (the outer layer of the integument) of hydrozoan coelenterates, the cell wall of fungi, and the cuticle of all arthropods. Aditional relevant polysaccharides are mannans, galactomannans, alginates, laminarins, carregeenans (iota and kappa), and agars.

Any enzyme capable of degrading any one or more of the above polymers, including collagen and/or cellulose and/or chitin can therefore be included into the coating composition of the invention, optionally in the absence of a substrate for such an enzyme, and preferably in combination with an oxidase, in the absence of a substrate for said oxidase.

The integument of most fouling organisms is the principal organ of permanent post-metamorphic attachment and adhesion. Interference with the synthesis of an important biochemical constituent of the cell wall or integument, or any degradation of such structural elements or interference with the enzymatic processes involved in adhesion would therefore exert a strong anti-fouling action.

As the bacterial and algae film production can well be a prerequisite for most macrofouling, this term refers to the attachment of organisms larger than unicellular organisms to an aquatic surface. Should this be the case, little or no enzyme or other chemical antifoulant capable of disrupting the attachment process of macrofouling organisms may need to be included as microfouling does not take place.

However, in a region that is heavily populated with barnacle larvae, enzymes which specifically retard the settlement of the barnacle larva would be more important and should be incorporated on a surface, preferably in larger proportion.

The coating compositions according to the invention in one embodiment result in the formation of essentially one or more monolayers of enzymes located on the surface of an object. For example, an enzyme having a molecular weight of approximately 50,000 daltons would give a monolayer when spaced on a surface with a distance of approximately 40 angstroms between the centers of adjacent molecules. This spacing assumes a Stokes radius of approximately 20 angstroms. However, it is not essential that a complete monolayer is present. A desirable activity can be maintained with the spacing of bioactive compounds over greater distances. A spacing of no more than 1,000 angstroms and more preferably no more than 100 angstroms is preferred in order to insure that a biologically active chemical is available for reaction with a fouling organism at each point of initial contact.

The coating compositions of the invention can be used in all types of environments, including non-aquatic as well as aquatic environemnts, including sea-water, estuary, and fresh water environments. In addition to natural environments (i.e., those which are in free contact with and freely exchange material with other parts of the biosphere without human intervention), the term "aquatic environments" as used herein also includes cooling towers, fresh and salt water piping systems, desalination and other filtration systems containing membrane "surfaces" subject to protection, and other aquatic environments which rely upon the intervention of human beings for their creation and maintenance.

As used herein, the term "natural environment" includes ponds, lakes, dredged channels and harbors, and other bodies of water which were initially produced by the action of human beings but which do not rely upon human intervention for the supply of water into and out of such environments.

While many fouling organisms, such as barnacles and algae, are well known to the general public, those skilled in the art will recognize that the term fouling organism as used herein refers to any living organism which is capable of attaching to a surface in an aquatic environment.

The group of algae are very diverse and probably not related to one another. There are 6 divisions of algae, some unicellular and some multicellular. In some taxonomic schemes, the last three divisions are included in the Kingdom Protista which includes all eukaryotic, unicellular organisms, regardless of their mode of nutrition.

Algae can be characterised with respect to e.g.:
1. Photosynthetic pigments. Some pigments mask the chlorophylls and give their name to the common name of the division - Brown algae. The accessory pigments participate with the PS II reaction center.
2. Food storage chemistry is an important distinguishing feature. Not all organisms store energy in the form of starch as do most plants. There are unique storage chemicals for the various division.
3. Flagella structure is a good distinguishing feature for those division that have flagellated cell. The number of flagella, morphology of the flagellum and its orientation characterize divisions.
4. Cell wall chemistry is another distinguishing feature.
5. Sometimes the habitat for members of the division can be important.

Rhodophyta are the red algae:
1. Pigments - the phycobolins, phycoerythrin and phycocyanin_are the pigments that usually mask the chlorophyll a that is common to all algae and the green plants.
2. Food storage materials - Floridean starch is a polysaccharide material.
3. Cell wall materials - The red algae possess a microfibrillar network of polysaccharide material (cellulose or some other) embedded within a mucilaginous matrix such as agar. Some marine forms may produce CaCO₃ in their walls to give them a rigid structure.
4. Types and number of flagella - The red algae never produce motile cells. Not only do they not produce motile cells, it appears that they may never have had motile cells.
5. Habitat - The red algae are mostly marine organisms but a few freshwater types do exist.
6. The life cycles of red algae are complicated by the presence of a third generation type in addition the sporophyte and gametophyte.

Phaeophyta are the brown algae. This group includes the kelps and rockweeds:
1. Pigments - The Brown algae have fucoxanthin as an accessory pigment to mask the chlorophyll a and c, giving them the brownish color.
2. Food storage materials - Lamanarin is a polysaccharide food storage material unique to the brown algae.
3. Cell wall materials include a mucilaginous material called algin that is harvested from kelps.
4. Types and number of flagella - The brown algae have heterokont flagellated cells. One is an anteriorly-oriented tinsel-type flagellum and the other flagellum is a posteriorly-oriented whiplash type.
5. Habitat - The brown algae are all marine organisms.
6. Several life cycle types are exemplified by the brown algae.
   - *Ectocarpus* is a filamentous alga that has an isomorphic alternation of generations.
   - *Laminaria* is a kelp that has a heteromorphic alternation of generations. The gametophyte is microscopic, whereas the sporophyte is macroscopic.
   - *Fucus* is a rockweed that has gametic meiosis. There is no alternation of generations for this organism. The gametangia, antheridia and oogonia, are produced within a conceptacle. Many conceptacles are located on a receptical at the end of the dichotomously branched thallus. Meiosis occurs in the production of the gametes.

Chlorophyta are the green algae. Because of the similarity in pigmentation, cell division, and food storage materials, the land plants are thought to be derived from the Chlorophyta:
1. Pigments - Chlorophyll b is the accessory pigment.
2. Food storage materials are starch.
3. Cell wall materials - are primarily cellulose but some marine forms may add CaCO₃.
4. Types and number of flagella of the chlorophyta are isokonts with whiplash flagella.
5. Habitat of chlorophyta is freshwater and marine.
6. Taxonomy of the chlorophyta is divided into three classes based on method of cell division, insertion of flagella and internal cell structure.
   - Method of cell division refers to the production of a phragmoplast. or a phy phycoplast.
   - Insertion of flagella are either apical or subapical.
   - Internal cell structure refers to the possession of a system of microtubules found near the flagella apparatus. Also the possession of peroxisomes involved in photoresiration.
7. Classes of Chlorophyta
   - Charophyceae are the group most like the land plants. They undergo mitosis by formation of a phragmoplast, possess the microtubular system characteristic of land plants, and have subapically inserted flagella. Example organisms in this group are *Spyrogyra,* the desmids and *Coleochaeta.*
   - The Ulvaphyceae are mostly marine organisms that have an alternation of generation. The life cycle of *Ulva* has an isomorphic alternation of generations with sporic meiosis. These organisms produce a phycoplast when undergoing cell division and the nuclear envelope persists during division.
   - Chlorophyceae produce a phycoplast when undergoing cell division and the nuclear envelope persists during division. There are many forms that have zygotic meiosis like *Chlamydamonas.*

Chrysophyta are unicellular algae:
1. Characteristics of the Chrysophyta indicate a similarity with the brown algae.
   There are three classes of chrysophyta.
   - Pigments include chlorophyll a and chlorophyll c. These are usually masked by an abundance of a brownish pigment, fucoxanthin.
   - Food reserve in the Chrysophyta is called chrysolaminarin - a carbohydrate.
   - The cell of chrysophytes may be naked or they may have cell walls of cellulose. Some members have silica scales or shells.
2. Classes of Chrysophyta
   - Chrysophyceae are primarily freshwater planktonic organisms. They lack a clearly defined cell wall but have silica scales. Many of these organisms have flagella.
   - Bacillariophyceae are the diatoms. These are important phytoplanktonic organisms in freshwater and marine environments. They are characterized by the presence of silica cell walls with intricate markings. They have chlorophyll a and c and fucoxanthin which gives them a brownish color. When they undergo sexual reproduction, the only flagellated cell appears, a males sperm cell. It has two flagella, one whiplash and one tinsel type.
   - Xanthophyceae are the yellow green algae because they lack fucoxanthin and the greenish colors show. Vaucheria, which you saw in lab belongs to this class.

Pyrrophyta are important phytoplanktonic organisms in freshwater and marine habitats:
1. Characteristics of Pyrrophyta
   - The dinoflagellates contain chlorophyll a and c and a brownish pigment called peridinin.
   - The food storage material of the pyrrophyta is starch.
   - The cell walls of those that possess them are in the form of cellulosic plates and hence the name armored dinoflagellates given to some members of the phylum.
   - The pyrrophyta have two flagella. One flagellum encircles the cell like a belt. The other flagellum trails behind the cell.
2. Features of the dinoflagellates
   - Some of these organisms are responsible for the poisonous red tide.
   - Some of these organisms are capable of bioluminescence.

Euglenophyta are unicellular algae that lack a cell wall:
1. Characteristics of the Euglenophyta
   - The euglenoids posses chlorophyll a and b and carotenoids. They have the same grass green color as the green algae.
   - The food storage material of the euglenoids is paramylon, a polysaccharide material
   - The euglenophyta lack cell walls. Instead they have a proteinaceous coating called the pellicle. They are capable of changing shape because they lack the cell wall.
   - The euglenoids have two flagella but only one flagellum emerges from a gullet at the tip of the cell. The other short flagellum is basically nonfunctional as a swimming aid.

Prevention and/or elimination or at least substantial reduction of microfouling by all or some of the above algae is within the scope of the present invention.

The term microfouling is used to denote the attachment of unicellular organisms, such as bacteria and algae, to the submerged surface. These microfouling organisms can, in some cases, secrete chemical signals which attract further organism to the surface, thereby increasing the rate of fouling. Macrofoulers, such as barnacles, become attached to the surface after the formation of the initial microfouling layer.

As microfouling may occur before the macrofouling, any process which interferes with the attachment of microbial organisms to aquatic surfaces would decrease the total amount of fouling which takes place. Thus, an active ingredient capable of preventing the attachment of barnacles operates at the end of the fouling chain while an active species which operates to prevent the attachment of unicellular organisms such as bacteria operates at the beginning of the fouling chain. Accordingly, species which prevent microfouling may have some inhibitory effect against settlement of all types of fouling. One such particularly preferred antifouling species, including an antimicrobial species is peroxides, such as hydrogen peroxide, produced by oxidases.

Additional antifouling organisms the growth of which is capable of being controlled by the means of the present invention as described herein includes, but is not limited to crustaceans and other marine hard growth, such as:
Tube Worms: polychaetes; phylum Annelida; subclass Eunicea; family Serpulidae
Mussels: bivalves; phylum Mollusca; subclass Pteriomorphia; family Mytilidae
Clams: bivalves; phylum Mollusca; subclass Hterodonta; family Veneridae
Bryozoans: bryozoans; phylum Bryozoa; suborder Anasca and Ascophora; genus Schizoporella
Barnacles: crustaceans; phylum Arthropoda; subphylum Crustacea

However, as is clear from the description herein above, the invention also has utility against soft growth, which can impede e.g. the efficiency of hull forms, damage substrates of marine structures, generally shorten the viable life span of equipment, and escalate the cost of operation. Examples of these soft growth forms include:
Algae (Botanus): Padina, and Codium
Bryozoans (Animal): Bugula Neretina
Hydroids (Animal): Obelia
Sabellids (Animal):
Delaya Marina (Marine Bacteria): Zibria

The compositions, coatings and/or paints according to the present invention may also function by direct attack on the surface film, disrupting its polymeric structure through e.g. hydrolysis of the proteins and polysaccharides of the film. This would interrupt the chain of events that ultimately leads to the accumulation of large amounts of marine organisms (including bacteria, fungi, barnacles, etc.) on e.g. the hull of the ship.

Such attack may be accomplished by the use of extracellular enzymes that disrupt the polysaccharides and proteins that make up the surface film. Key hydrolytic enzymes in this respect are proteases, alpha-amylases, amyloglycosidases and xylanases. Alternatively, the coatings and/or paints may function by modifying the surface tension of the marine surface to which the coatings and/or paints have been applied. Such a change in the surface tension may disrupt the colonization of the surface by undesirable marine organisms.

The methods and compositions disclosed herein may be used on a variety of surfaces, including but not limited to boat hulls, marine markers, bulkheads, pilings, water inlets, floors, roofs, and shingles. For example, the methods and compositions may be used to minimize fouling of marine markers. Such markers constitute a large category of floating objects and are greatly impaired by the accumulation of marine growth.

Similarly, the methods and compositions may be used on marine bulkheads. The accumulation of marine growth on bulkhead structures is detrimental to the bulkhead structure over the long term. Furthermore, the growth causes significant short term effects that are aesthetically displeasing and dangerous. Moreover, the harsh abrasive characteristics of the hard growth can result in major damage to vessels.

Similarly, the present invention can be used to minimize blockages due to fouling by marine growth of heat exchangers, evaporators, condensers and fire and flushing systems, thus resulting in significant decreases in maintenance costs for all categories of marine structures.

Compositions and/or paints according to the invention may include various hydrolytic enzymes, although it is possible to practice the invention without such hydrolytic enzymes. Examples of suitable enzymes include proteases, including subtilisins such as e.g. alcalase, amylases, amyloglycosidases, xylanases and other hydrolytic enzymes known in the art. The hydrolytic enzymes selected should act to prevent or reduce attachment by unwanted or undesirable marine organisms. The hydrolytic enzymes chosen should be able to survive and flourish in the marine environment to which they will be exposed.

Compositions and/or paints according to the invention include the above-mentioned enzymes in an amount effective to reduce the growth of unwanted or undesirable microorganisms. Such compositions and/or paints may be in a variety of forms, including paints, lacquers, pastes, laminates, epoxies, resins, waxes, gels, and glues in addition to other forms known to one of skill in the art.

The compositions and/or paints may be polymeric, oligomeric, monomeric, and may contain cross-linkers or cure promoters as needed. Such compositions and/or paints may contain other additives, in addition to those mentioned above, to accomplish purposes known to one of skill in the art. Such other additives include preservatives, pigments, dyes, fillers, surfactants, and other additives known to one of skill in the art.

### Selected antifouling species

Peroxides in general constitute one much preferred group of antifouling species, including an antimicrobial species according to the invention. Hydrogen peroxide is an example of a presently most preferred antifouling species, including an antimicrobial species.

Any enzyme-compound combination capable of producing hydrogen peroxide can be used, including a combination wherein the enzyme is an oxidase and the compound can be oxidized by said oxidase.

A combination of said oxidase with said compounds to be oxidized thereby includes such combinations as (enzyme-substrate) malate oxidase-malic acid; glucose oxidase-glucose; hexose oxidase-glucose; cholesterol oxidase-cholesterol; arylalcohol oxidase-arylalcohol: galactose oxidase-galactose; alcohol oxidase-alcohol; lathosterol oxidase-lathosterol; aspartate oxidase-aspartic acid; L-amino-acid oxidase-L-amino acid; D-amino-acid oxidase-D-amino acid; amine oxidase-amine; D-glutamate oxidase-glutamine; ethanolamine oxidase-ethanolamine; NADH oxidase-NADH; urate oxidase (uricase)-uric acid; superoxide dismutase-superoxide radical; and so forth.

The enzymatic reaction between said oxidase and the compound yields hydrogen peroxide. The enzymatic reaction can proceed when either oxygen or oxygen and water are present in an external environment contacting the coating composition according to the invention.

The above-mentioned oxygen is supplied not only from atmospheric air but also from e.g. seawater containing dissolved oxygen. The enzymatic reaction of the invention occurs in an external environment including seawater with the result that hydrogen peroxide is produced in said environment.

Additional preferred species having antimicrobial activity includes, but is not limited to, carboxyl group-containing species, hydroxyl group-containing species, amino group-containing species, aldehyde group-containing species, and decomposition products of chitosan.

The carboxyl group-containing species includes a variety of organic acid species, e.g. aliphatic acids such as formic acid, acetic acid, propionic acid, butyric acid, caproic acid, caprylic acid, capric acid, lauric acid, myristic acid, palmitic acid, oleic acid, linoleic acid, linolenic acid, monochloroacetic acid, monofluoroacetic acid, sorbic acid, undecylenic acid, etc.; dibasic acids such as oxalic acid etc.; aromatic carboxylic acids such as benzoic acid, p-chlorobenzoic acid, p-hydroxybenzoic acid, salicylic acid, cinnamic acid, etc.; and their derivatives and halides. Any enzyme-compound combination capable of producing a carboxyl group-containing species can be applied.

The ester bond-containing species mentioned above is not particularly restricted in kind but includes, among others, esters of any of said carboxyl group-containing species with aliphatic alcohols such as methyl alcohol, ethyl alcohol, propyl alcohol, butyl alcohol, pentyl alcohol, caproyl alcohol, caprylyl alcohol, capryl alcohol, lauryl alcohol, myristyl alcohol, palmityl alcohol, oleyl alcohol, etc.; esters of any of said carboxyl group-containing species with aromatic alcohols such as phenol, benzyl alcohol, etc.; esters of any of said carboxyl group-containing species with polyhydric alcohols such as ethylene glycol, glycerol, etc.; and esters of any of said carboxyl group-containing species with derivatives or halides of said aliphatic alcohols, aromatic alcohols, or polyhydric alcohols.

The ester bond-containing species mentioned above is hydrolyzed by said esterase in the above-mentioned coating composition to produce said carboxylic group-containing species. This enzymatic reaction can proceed when water is present in the reaction system, as follows.

R₁COOR₂ + H₂O => R₁COOH + R₂OH

In the above reaction scheme, R₁ represents carboxylic residue and R₂ represents an alcohol residue.

When the above coating composition is applied to an object, the antimicrobial effect is achieved when e.g. moisture from the atmosphere is provided to the reaction resulting in the production of an antifouling species, including an antimicrobial species. When the coating composition is applied to an object to be placed in an aqueous environemnt e.g. in water such as seawater, the reaction resulting in the production of antifouling species, including an antimicrobial species takes place in said water.

The amide bond-containing species mentioned above includes, but is not limited to, amides of any of said carboxyl group-containing species with aliphatic amines such as butylamine, hexylamine, octylamine, decylamine, laurylamine, stearylamine, oleylamine, etc.; and amides of any said carboxyl group-containing species with aromatic amines such as aniline, toluidine, xylidine, and alkylanilines such as hexylaniline, octylaniline, nonylaniline, dodecylaniline, and so forth.

The hydroxyl group-containing species mentioned above includes, but is not limited to, aliphatic alcohols such as methyl alcohol, ethyl alcohol, propyl alcohol, isopropyl alcohol, butyl alcohol, isobutyl alcohol, pentyl alcohol, isopentyl alcohol, hexyl alcohol, etc.; aromatic alcohols such as phenol, chlorophenol, and alkylphenols such as cresol, xylenol, etc., resorcinol, benzyl alcohol, etc.; and the derivatives and halides of said aliphatic or aromatic alcohols.

Any enzyme-compound combination capable of producing the hydroxyl group-containing species can be applied. In one embodiment, the enzyme is an esterase and the compound is an ester bond-containing species. The esterase and the ester bond-containing species includes the species mentioned hereinbefore, but is not limited to these species.

The amino group-containing species mentioned above includes, but is not limited to aliphatic amines such as butylamine, hexylamine, octylamine, decylamine, laurylamine, stearylamine, oleylamine, cyclohexylamine, etc.; and aromatic amines such as aniline, toluidine, xylidine, p-n-hexylaniline, p-n-octylaniline, p-nonylaniline, p-dodecylaniline, and so forth.

Any enzyme-compound combination capable of producing said amino group-containing species can be used. Preferred is the case in which the enzyme is an amidase including a protease, and the compound is an amide bond-containing species including apolypeptide. The amidase and the amide bond-containing species includes the species mentioned hereinbefore, but is not limited to these species.

The aldehyde group-containing species includes, but is not limited to aliphatic aldehydes such as formaldehyde, glyoxal, succinaldehyde, glutaraldehyde, capronaldehyde, caprylaldehyde, caprinaldehyde, laurinaldehyde, stearinaldehyde, oleinaldehyde, etc.; benzaldehyde and its derivatives such as p-n-hexylbenzaldehyde, p-octylbenzaldehyde, p-oleylbenzaldehyde, vaniline, piperonal, etc.; salicylaldehyde, cinnamaldehyde, and so forth.

Any enzyme-compound combination capable of producing said aldehyde group-containing species can be used, including the case in which the enzyme is alcohol dehydrogenase and the compound is an aliphatic alcohol, e.g. methanol, ethanol, etc.; the case in which the enzyme is alcohol oxidase and the compound is an aliphatic alcohol such as methanol, ethanol, etc.; the case in which the enzyme is arylalcohol dehydrogenase and the compound is an aromatic alcohol such as phenol, cresol, etc.; and the case in which the enzyme is amine oxidase and the compound is an aliphatic amine such as butylamine, hexylamine, and so forth.

Any enzyme-compound combination capable of producing a decomposition product of chitosan can be applied. Preferred is the case in which the enzyme is a chitosan-decomposing enzyme and the compound is chitosan.

### Enzyme concentrations

In one preferred embodiment the aerogel comprises from 1 to 90 weight% enzyme, such as from 1 to 85 weight%, for example from 1 to 80 weight%, such as from 1 to 75 weight%, for example from 1 to 70 weight%, such as from 1 to 65 weight%, for example from 1 to 60 weight%, such as from 1 to 55 weight%, for example from 1 to 50 weight%, such as from 1 to 45 weight%, for example from 1 to 40 weight%, such as from 1 to 35 weight%, for example from 1 to 30 weight%, such as from 1 to 25 weight%, for example from 1 to 20 weight%, such as from 1 to 25 weight%, for example from 1 to 20 weight%, such as from 1 to 15 weight%, for example from 1 to 10 weight%, such as from 1 to 5 weight%, for example from 1 to 4 weight%, such as from 1 to 4 weight%, for example from 1 to 3 weight%, such as from 1 to 2 weight%, for example from 5 to 90 weight%, such as from 10 to 90 weight%, for example from 15 to 90 weight%, such as from 20 to 90 weight%, for example from 25 to 90 weight%, such as from 30 to 90 weight%, for example from 35 to 90 weight%, such as from 40 to 90 weight%, for example from 45 to 90 weight%, such as from 50 to 90 weight%, for example from 55 to 90 weight%, such as from 60 to 90 weight%, for example from 65 to 90 weight%, such as from 70 to 90 weight%, for example from 75 to 90 weight%, such as from 80 to 90 weight%, for example from 85 to 90 weight%, such as from 1 to 5 weight%, for example from 5 to 10 weight%, such as from 10 to 15 weight%, for example from 15 to 20 weight%, such as from 20 to 25 weight%, for example from 25 to 30 weight%, such as from 30 to 35 weight%, for example from 35 to 40 weight%, such as from 40 to 45 weight%, for example from 45 to 50 weight%, such as from 50 to 55 weight%, for example from 55 to 60 weight%, such as from 60 to 65 weight%, for example from 65 to 70 weight%, such as from 70 to 75 weight%, for example from 75 to 80 weight%, such as from 80 to 85 weight%, for example from 85 to 90 weight%.

One embodiment relates to a method for increasing the amount of a bioactive agent in a coating composition comprising an organic solvent, said method comprising the steps of:
a) providing a coating composition comprising an organic solvent,
b) providing an aerogel comprising one or more biologically active enzymes entrapped therein,
c) mixing the coating composition provided in step a) with the aerogel comprising one or more biologicaly enzymes entrapped therein as provided in step b), thereby
d) obtaining a coating composition comprising an organic solvent and an aerogel comprising one or more biologically active enzymes entrapped therein,
wherein said one or more biologically active enzymes are present in a higher concentration and/or possesses a higher biological activity than would have been the case had said one or more enzymes not been entrapped in said aerogel structure

### Additional components of coating compositions of the invention

In one preferred embodiment the anti-fouling composition comprising an aerogel further comprises at least one algicide, herbicide, fungicide, molluscicide or other compound exhibiting anti-fouling activity.

In one preferred embodiment the anti-fouling composition comprising an aerogel further comprises a binder component, suitable for marine applications and a pigment.

The coating compositions of the invention described herein above can further comprise a binder to immobilise at least one of the constituents, optionally to immobilise the enzymes.

The coating compositions of the present invention can be formulated as coatings, lacquers, stains, enamels and the like, hereinafter referred to generically as "coating (s)".

Preferably, the coating composition is formulated for treatment of a surface selected from outdoor wood work, external surface of a central heating system, and a hull vehicle should not interfere with the activity of the at least one enzyme(s) and/or any additional antifoulant compound.

Suitable solvents for coating compositions are disclosed e.g. in US 5,071,479 and include water and organic solvents including aliphatic hydrocarbons, aromatic hydrocarbons, such as xylene, toluene, mixtures of aliphatic and aromatic hydrocarbons having boiling points between 100°C and 320°C, preferably between 150°C and 230°C; high aromatic petroleum distillates, e. g., solvent naptha, distilled tar oil and mixtures thereof ; alcohols such as butanol, octanol and glycols; yegetable and mineral oils; ketones such as acetone; petroleum fractions such as mineral spirits and kerosene, chlorinated hydrocarbons, glycol esters, glycol ester ethers, derivatives and mixtures thereof.

The solvent may be apolar or polar, such as water, optionally in admixture with an oily or oil-like low-volatility organic solvent, such as the mixture of aromatic and aliphatic solvents found in white spirits, also commonly called mineral spirits.

The solvent may typically contain at least one of a diluent, an emulsifier, a wetting agent, a dispersing agent or other surface bioactive agent. Examples of suitable emulsifiers are disclosed in US-A-5071479 and include nonylphenol-ethylene oxide ethers, polyoxyethylene sorbitol esters or polyoxyethylene sorbitan esters of fatty acids, derivatives and mixtures thereof.

Any suitable surface coating material may be incorporated in the composition and/or coating of the present invention. Examples of trade-recognized coating materials are polyvinyl chloride resins in a solvent based system, chlorinated rubbers in a solvent based system, acrylic resins and methacrylate resins in solvent based or aqueous systems, vinyl chloride-vinyl acetate copolymer systems as aqueous dispersions or solvent based systems, butadiene copolymers such as butadiene-styrene rubbers, butadiene-acrylonitrile rubbers, and butadiene-styrene-acrylonitrile rubbers, drying oils such as linseed oil, alkyd resins, asphalt, epoxy resins, urethane resins, polyester resins, phenolic resins, derivatives and mixtures thereof.

The composition and/or coating of the present invention may contain pigments selected from inorganic pigments, such as titanium dioxide, ferric oxide, silica, talc, or china clay, organic pigments such as carbon black or dyes insoluble in sea water, derivatives and mixtures thereof.

The coating composition of the present invention can also contain plasticisers, rheology characteristic modifiers, other conventional ingredients and mixtures thereof.

The coating composition of the present invention optionally further comprise an adjuvant conventionally employed in compositions used for protecting materials exposed to an aquatic environment. These adjuvants may be selected from additional fungicides, auxiliary solvents, processing additives such as defoamers, fixatives, plasticisers, UV-stabilizers or stability enhancers, water soluble or water insoluble dyes, color pigments, siccatives, corrosion inhibitors, thickeners or antisettlement agents such as carboxymethyl cellulose, polyacrylic acid or polymethacrylic acid, anti-skinning agents, derivatives and mixtures thereof.

In one embodiment the present invention provides a marine anti-foulant comprising the coating composition as described above. Preferably, the anti-foulant is self-polishable.

According to the present invention, the enzyme is encapsulated, such as encapsulated by a semi-permeable membrane. One type of enzymes may be encapsulated individually independently of other types of enzymes, or the enzymes may be encapsulated together. The encapsulating material may be selected such that on contact with a foulant, the enzyme may be released. In this way, a composition may be provided which only provides an anti-foulant species or increases provision of an anti-foulant compound when contacted with a foulant. Alternating layers of anti-foulant species and encapsulation material ensures a sequential release of enzymes.

The composition of the present invention can be provided as a ready-for-use product or as a concentrate. The ready-for-use product may be in the form of an aqueous solution, aqueous dispersion, oil solution, oil dispersion, emulsion, or an aerosol preparation. The concentrate can be used, for example, as an additive for coating, or can be diluted prior to use with additional solvents or suspending agents.

An aerosol preparation according to the invention may be obtained in the usual manner by incorporating the composition of the present invention comprising or dissolved or suspended in, a suitable solvent, in a volatile liquid suitable for use as a propellant.

As discussed in US 5,071,479, the coating composition of the present invention can also include additional ingredients known to be useful in preservatives and/or coatings. Such ingredients include fixatives such as carboxymethylcellulose, polyvinyl alcohol, paraffin, co-solvents, such as ethylglycol acetate and methoxypropyl acetate, plasticisers such as benzoic acid esters and phthlates, e. g., dibutyl phthalate, dioctyl phthalate and didodecyl phthalate, derivatives and mixtures thereof. Optionally dyes, color pigments, corrosion inhibitors, chemical stabilizers or siccatives (dryers) such as cobalt octate and cobalt naphthenate, may also be included depending on specific applications.

The composition and/or coating of the present invention can be applied by any of the techniques known in the art including brushing, spraying, roll coating, dipping and combinations thereof.

Compositions of the present invention can be prepared simply by mixing the various ingredients at a temperature at which they are not adversely affected. Preparation conditions are not critical. Equipment and methods conventionally employed in the manufacture of coating and similar compositions can be advantageously employed.

### Paint

In another embodiment the aerogel comprising one ore more bioactive agents is used as an agent in a paint or any other preservation for protection of any type of surface such as wood, metal, stone, bricks, concrete and plastic. The present invention e.g. relates to house painting and wood or metal protection.

In one preferred embodiment the paint or other type of coating composition comprices aerogel particles.

In one preferred embodiment the paint or other type of coating composition comprices one or more premade aerogel(s). After mixing of the one or more aerogels with the paint or other type of coating composition the physical and chemical properties of the one or more aerogels may be affected.

In one preferred embodiment an antifouling paint composition comprises at least one subtilisin (EC 3.4.21.62), said subtilisin having the following characteristica: (i) optimum activity at a pH in the range of about 7 - 10, and (ii) optimum activity at a temperature in the range of about 55 - 65°C. In another embodiment the antifouling paint composition comprises the subtilisin Alcalase². In one preferred embodiment the antifouling paint composition comprises the Alcalase^{®} Alcalase 2.5 L, Type DX^{®}.

Paint is any liquid, liquifiable, or mastic composition which after application to a surface in a thin layer is converted to an opaque solid film.

The paint or preservative is used to protect, decorate (such as adding color), or add functionality to an object or surface by covering it with a pigmented coating. An example of protection is to retard corrosion of metal. An example of decoration is to add festive trim to a room interior. An example of added functionality is to modify light reflection or heat radiation of a surface. Another example of functionality would be the use of color to identify hazards or function of equipment and pipelines.

Paint can be applied to almost any kind of object. It is used, among many other uses, in the production of art, in industrial coating, as a driving aid (road surface marking), or as a barrier to prevent corrosion or water damage. Paint is a semifinished product, or intermediate good as the final product is the painted article itself.

Paint can also be mixed with glaze to create various textures and patterns. This process is referred to as faux finish.

The present invention also relates to in-can preservation of paint.

### Components of paint

There are three primary components to a paint:
1) Pigments;
2) Binder, also known as non-volatile vehicle or resin and
3) Vehicle, also known as volatile vehicle, also called solvent.

### Pigment

Pigments impart such qualities as color and opacity, and influence properties such as gloss, film flow, and protective abilities. Pigment can generally be categorized into two main types: Prime or hiding pigments and Inert or extender pigments.

The main modern white hiding pigment is Titanium dioxide. Zinc oxide is a weaker white pigment with some important usages. Color hiding pigments fall also into two main categories, those being Inorganic, mostly duller earth tone colors, and Organic, generally brighter but more expensive colors.

Inert pigments break down into natural or synthetic types. Natural pigments include various clays, calcium carbonate, mica, silicas, and talcs. Synthetics would include calcined clays, blanc fix, precipitated calcium carbonate, and synthetic silicas.

Hiding pigments, in making paint opaque, also protect the substrate from the harmful effects of ultraviolet light.

Some pigments are toxic, such as the lead pigments that are used in lead paint. Paint manufacturers began replacing white lead pigments with the less toxic substitute, which can even be used to color food, titanium white (titanium dioxide), even before lead was functionally banned in paint for residential use in 1978 by the U.S. Consumer Product Safety Commission.

Titanium dioxide was first used in paints in the 19th century. The titanium dioxide used in most paints today is often coated with silicon or aluminum oxides for various reasons such as better exterior durability, or better hiding performance (opacity) via better efficiency promoted by more optimal spacing within the paint film. Opacity is also improved by optimal sizing of the titanium dioxide particles.

The present invention relates to an antifouling coating composition comprising one ore more aerogels, and one or more bioactive agents and further comprising any pigment described in the prior art or any combination thereof.

### Binder

The binder, or resin, is the actual film forming component of paint. It imparts adhesion, binds the pigments together, and strongly influences such properties as gloss potential, exterior durability, flexibility, and toughness.

Binders include synthetic or natural resins such as acrylics, polyurethanes, polyesters, melamine resins, epoxy, or oils.

Binders can be categorized according to drying, or curing, mechanism. The four most common are simple solvent evaporation, oxidative crosslinking, catalyzed polymerization, and coalescence.

Note that drying and curing are two different processes. Drying generally refers to evaporation of vehicle, whereas curing refers to polymerization of the binder. Depending on chemistry and composition, any particular paint may undergo either, or both processes. Thus, there are paints that dry only, those that dry then cure, and those that do not depend on drying for curing.

Paints that dry by simple solvent evaporation contain a solid binder dissolved in a solvent; this forms a solid film when the solvent evaporates, and the film can redissolve in the solvent again. Classic nitrocellulose lacquers fall into this category, as do non-grain raising stains composed of dyes dissolved in solvent.

Paints that cure by oxidative crosslinking are generally single package coatings that when applied, the exposure to oxygen in the air starts a process that crosslinks and polymerizes the binder component. Classic alkyd enamels would fall into this category.

Paints that cure by catalyzed polymerization are generally two package coatings that polymerize by way of a chemical reaction initiated by mixing resin and hardener, and which cure by forming a hard plastic structure. Depending on composition they may need to dry first, by evaporation of solvent. Classic two package epoxies or polyurethanes would fall into this category.

Latex paints cure by a process called coalescence where first the water, and then the trace, or coalescing, solvent, evaporate and draw together and soften the latex binder particles together and fuse them together into irreversibly bound networked structures, so that the paint will not redissolve in the solvent/water that originally carried it.

Recent environmental requirements restrict the use of Volatile Organic Compounds (VOCs), and alternative means of curing have been developed, particularly for industrial purposes. In UV curing paints, the solvent is evaporated first, and hardening is then initiated by ultraviolet light. In powder coatings there is little or no solvent, and flow and cure are produced by heating of the substrate after application of the dry powder.

The present invention relates to an antifouling coating composition comprising one ore more aerogels, and one or more bioactive agents and further comprising any binder described in the prior art or any combination thereof.

### Vehicle, or solvent

The main purpose of the vehicle is to adjust the viscosity of the paint. It is volatile and does not become part of the paint film. It can also control flow and application properties. Its main function is as the carrier for the non volatile components.

Water is the main vehicle for water based paints.

Solvent based, sometimes called oil based, paints can have various combinations of solvents as the vehicle, including aliphatics, aromatics, alcohols, and ketones. These include organic solvents such as petroleum distillate, alcohols, ketones, esters, glycol ethers, and the like. Sometimes volatile low-molecular weight synthetic resins also serve as diluents.

The present invention relates to an antifouling coating composition comprising one ore more aerogels, and one or more bioactive agents and further comprising any vehicle or solvent described in the prior art or any combination thereof.

### Additives

Besides the three main categories of ingredients, paint can have a wide variety of miscellaneous additives, usually added in very small amounts. Some examples include additives to improve wet edge, improve pigment stability, impart antifreeze properties, control foaming, control skinning, etc. Other additives might be thickeners, coalescent solvents, or biocides to fight bacterial growth.

The present invention relates to an antifouling coating composition comprising one ore more aerogels, and one or more bioactive agents and further comprising any coating composition additives described in the prior art or any combination thereof.

Fillers serve to thicken the film, support its structure and simply increase the volume of the paint. Not all paints include fillers. Pigments that also function as fillers are called simply "pigments"; "fillers" are generally color-neutral and opaque. It is necessary to adjust the resulting off-white color with pigments to give the desired color. Common fillers are cheap and inert, such as talc, lime, baryte, clay, etc. Depending on the paint, most of the paint film may consist of pigment/filler and binder, the rest being other additives.

Besides pigments and dyes, other types of additives include catalysts, thickeners, stabilizers, emulsifiers, texturizers, adhesion promoters, flatteners (de-glossing agents), and the like.

After application, the paint solidifies and becomes tack-free. Depending on the type of binder, this hardening may be a result of curing (polymerization), evaporation, or even phase change brought about by cooling. In oil-based paint, curing takes the form of oxidation, for example oxidation of linseed oil to form linoxin to create a varnish. Other common cured films are prepared from crosslinkers, such as polyurethane or melamine resins, reacted with acrylic polyester or polyurethane resins, often in the presence of a catalyst which serves to make the curing reaction proceed more quickly or under milder conditions. These cured-film paints can be either solvent-borne or waterborne.

Latex paint is a water-based dispersion of sub-micron polymer particles. The term "latex" in the context of paint simply means an aqueous dispersion; latex rubber (the sap of the rubber tree that has historically been called latex) is not an ingredient. These dispersions are prepared by emulsion polymerization. When the water evaporates, the polymer particles coalesce to form a solid film. The polymer itself resists water (and typically some other solvents). Residual surfactants in the paint as well as hydrolytic effects with some polymers cause the paint to remain susceptible to softening and, over time, degradation by water.

Still other films are formed by cooling of the binder. For example, encaustic or wax paints are liquid when warm, and harden upon cooling.

The present invention relates to an antifouling coating composition comprising one ore more aerogels, and one or more bioactive agents and further comprising any miscellaneous coating composition agent described in the prior art or any combination thereof.

### Product variants

- Primer is a preparatory coating put on materials before painting. Priming ensures better adhesion of paint to the surface, increases paint durability, and provides additional protection for the material being painted.
- Varnish and shellac provide a protective coating without changing the color. They are paints without pigment.
- Wood stain is a type of paint that is very "thin," that is, low in viscosity, and formulated so that the pigment penetrates the surface rather than remaining in a film on top of the surface. Stain is predominantly pigment or dye and solvent with little binder, designed primarily to add color without providing a surface coating.
- Lacquer is usually a fast-drying solvent-based paint or varnish that produces an especially hard, durable finish.
- An enamel paint is a paint that dries to an especially hard, usually glossy, finish. Enamel can be made by adding varnish to oil-based paint.
- A Glaze is an additive used with paint to slow drying time and increase translucency, as in Faux Painting and Art Painting.
- A Roof coating is a fluid applied membrane which has elastic properties that allows it to stretch and return to their original shape without damage. It provides UV protection to polyurethane foam and is widely used as part of a roof restoration system.
- Fingerpaint
- Inks are similar to paints, except they are typically made using dyes exclusively (no pigments), and are designed so as not to leave a thick film of binder.
- Titanium dioxide is extensively used for both house paint and artist's paint, because it is permanent and has good covering power. Titanium oxide pigment accounts for the largest use of the element. Titanium paint is an excellent reflector of infrared, and is extensively used in solar observatories where heat causes poor seeing conditions.
- Anti-Graffiti paints are used to defeat the marking of surfaces by graffiti artists. There are two categories, sacrificial and non-bonding. Sacrificial coatings are clear coatings that allow the removal of graffiti, usually by pressure washing the surface with high-pressure water, removing the graffiti, and the coating (hence, sacrificed.) They must be re-applied afterward for continued protection. This is most commonly used on natural-looking masonry surfaces, such as statuary and marble walls, and on rougher surfaces that are difficult to clean. Non-bonding coatings are clear, high-performance coatings, usually catalyzed polyurethanes, that allow the graffiti very little to bond to. After the graffiti is discovered, it can be removed with the use of a solvent wash, without damaging the underlying substrate or protective coating. These work best when used on smoother surfaces, and especially over other painted surfaces, including murals.
- Anti-climb paint is a non-drying paint that appears normal while still being extremely slippery. It is usually used on drainpipes and ledges to deter burglars and vandals from climbing them, and is found in many public places. When a person attempts to climb objects coated with the paint, it rubs off onto the climber, as well as making it hard for them to climb.
- No-VOC paints, which are solvent-free paints that do not contain volatile organic compounds, have been available since the late 1980s. Low VOC paints, which typically contain anywhere between 0.3%-5.0% VOCs as coalescent, or coalescing solvent have been available since the 1960s.

### Preferred methods and uses

Preferred uses of the present invention include the following methods, but is not limited thereto:

Method for treating a surface contacted by fouling organisms, or a surface at risk of such contact, said method comprising the steps of contacting the surface with a composition according to the invention with an effective amount of said composition or coating composition, wherein said contacting results in eliminating said fouling or at least reducing said fouling.

Method for preventing or reducing fouling of a surface, said method comprising the steps of contacting the surface with a composition according to the invention with an effective amount of said composition or coating composition or hygienic composition, wherein said contacting results in preventing or reducing fouling of said surface.

Method for treating a surface contacted by a fluid composition comprising fouling organisms, said method comprising the steps of contacting the surface with a composition according to the invention with an effective amount of said composition or coating composition, wherein said contacting prevents fouling of said surface, or results in a reduced fouling of said surface.

The above-mentioned surfaces can be at least partly submerged in seawater, or they can be interior or exterior surfaces of a pipe for ventilation, or interior walls in a building.

Additional methods are:
Method for disinfecting a surface, said method comprising the steps of contacting the surface with a composition according to the invention with an effective amount of said composition or coating composition or hygienic composition, wherein said contacting results in a disinfection of said surface.
Method for removing microbial organisms from a surface, said method comprising the steps of contacting the surface with a composition according to the invention with an effective amount of said composition or coating composition or hygienic composition, wherein said contacting results in removing microbial organisms from said surface.
Method for coating an object, said method comprising the steps of contacting the surface with a composition according to the invention with an effective amount of said composition or coating composition or hygienic composition, wherein said contacting results in coating said object.
Method for sealing a surface, said method comprising the steps of contacting the surface with a composition according to the invention with an effective amount of said composition or coating composition or hygienic composition, wherein said contacting results in sealing said surface from an external environment.
Method for reducing or eliminating marine corrosion, said method comprising the steps of contacting the surface with a composition according to the invention with an effective amount of said composition or coating composition or hygienic composition, wherein said contacting results in reducing or eliminating marine corrosion.
Method for preserving a surface, said method comprising the steps of contacting the surface with a composition according to the invention with an effective amount of said composition or coating composition or hygienic composition, wherein said contacting results in preserving said surface.
Method for killing undesirable microbial cells, said method comprising the steps of contacting the surface with a composition according to the invention with an effective amount of said composition or coating composition or hygienic composition, wherein said contacting results in killing undesirable microbial cells.
Method for generating an antifouling species, said method comprising the steps of providing a composition comprising at least one enzyme capable of acting on a compound, wherein said action results in the formation of an antifouling species comprising an antifouling activity, wherein said compound does not form part of said composition, further providing said compound, and forming said antifouling species by contacting said at least one enzyme with said compound.
Method for preparing a painting composition, said method comprising the steps of providing at least one pigment and at least one enzyme capable of acting on a compound, wherein said action results in the formation of an antifouling species comprising an antifouling activity, wherein said compound does not form part of said composition, further providing a carrier for said at least one enzyme, and forming said composition by contacting said at least one enzyme with said carrier.

Preferred uses include, but is not limited to:
Use of at least one enzyme comprising an oxidase activity in the manufacture of a coating composition, wherein said coating composition does not comprise any substrate for said oxidase activity.
Use of at least one enzyme comprising an oxidase activity in a cleaning in place system, wherein said system does not comprise any substrate for said oxidase.

The aerogel may be used at the vehicle to distribute proteins in a hydrophobic phase in a molecular disperse distribution not achievable by other means.

The aerogel may be used to influence the rate of polishing for a film prepared from the mixture

The aerogel can be used to influence the viscosity of the film forming mixture to optimize film formation

In another preferred embodiment the aerogel material described is used for thermal insulation material.

In another embodiment the aerogel is used as a chemical absorber for cleaning up spills. The aerogel can also be used as a catalyst or a catalyst carrier.

In another embodiment the aerogel is used as an agent in cosmetics.

Resorcinol-formaldehyde aerogels (polymers chemically similar to phenol formaldehyde resins) are mostly used as precursors for manufacture of carbon aerogels, or when an organic insulator with large surface is desired. They come as high-density material, with surface area about 600 m²/g.

An embodiment concerns an antifouling coating composition, for instance a painting composition, for use in the prevention, reduction or removal of fouling of a surface or material. In an embodiment said surface or material has been submerged in water, such as sea water or fresh water, and has been fouled by fouling organisms during said submersion in water. In an embodiment the composition comprises an aerogel. In a further embodiment said aerogel comprises at least one enzyme, such as esterase.

The methods may in an embodiment be employed in the removal, reduction or prevention of fouling resulting from submersion of a material in water, such as seawater or fresh water.

The methods and composition are in an embodiment particularly suitable for use in the reduction, prevention or removal of fouling of materials or surfaces having been submerged in seawater or fresh water, such as for instance ship hulls, buoys or other structures being exposed to water.

It is a particular advantage that the compositions and methods are capable of effectively preventing fouling without the use of toxic substances.

### Examples

### Example 1 (not according to the present invention)

The aerogel comprises siliciumoxid. The aerogel is obtained by hydrolysis of tetraalkoxysiloxane dissolved in alcohol. The alcohol is subsequently removed from the generated network by exchange with supercritical CO2. This solvent can be evaporated without collaps of the aerogel.

The aerogel is obtained by drying of a wet gel. During this process the liquid is removed from the nanopores in the gel. The drying is performed in the presence of supercritical CO2 or another supercritical solvent. It is important to go directly from the liquid phase to a supercritical phase and subsequently directly from the supercritical phase to a gas phase. An alternative to the supercritic drying is use of DCCA (Drying Control Chemical Additives), ambient pressure drying and freeze drying.

The wet gel is obtained by a SOL-GEL process. The SOL-GEL process is typically performed at temperatures lower than 100°C. For preparation of in-organic gells, typically metaloxides, the reaction typically occur by condensation of metalhydroxides in solution such as they are obtained by hydrolysis of one or more metalalkoxides. In the first step (referred to as SOL) small independent more or less cross-linked colloid particles are performed in a colloid suspension. In the second step (referred to as GEL) these colloid particles bind to each other. This three-dimensional network is known as GEL. If the three-dimensional network is generated from linear polymer chains from the precursor solution, without a preference for individual particles, a polymer gel is obtained.

### Example 2

The aerogel is obtained as described in example 1. The aerogel further comprises one or more enzymes and/or one or more other bioactive agents. The aerogel which comprises one or more enzyme(s) as well as the aerogel which comprises one or more enzyme(s) and one or more other bioactive agent(s) are according to the present invention. The one or more enzymes and/or the one or more other bioactive agents are added to the alcohol during the generation of the network. This process results in encapsulation of the one or more enzymes and/or the one or more other bioactive agents in the aerogel. The process descibed in example 1 may be interrupted after preparation of colloid particles containing an enzyme as descibed in example 2. Completion of the network may be performed under addition of one or more different active components. Hereby the control of spatically distributed actiove compounds can be obtained

### Example 3

The aerogel is obtained as described in example 1 or 2. The aerogel which comprises one or more enzyme(s) as well as the aerogel which comprises one or more enzyme(s) and one or more other bioactive agent(s) are according to the present invention. The aerogel further comprises 1-10% dimethyldialkoxysiloxane to adjust the hydrophobicity of the aerogel. Other alkoxysiloxanes may me used to obtain similar adjustments in hydrophobicity.

### Example 4

The aerogel is obtained as describes in example 1, 2 or 3. The aerogel which comprises one or more enzyme(s) as well as the aerogel which comprises one or more enzyme(s) and one or more other bioactive agent(s) are according to the present invention. Si is completely or partly replaced with Ti, Al or Boron. This replacement affects the stability of the aerogel. The replacement can be introduced statistically. It is possible by prepreparation of colloid particles to obtain a spatically inhomogeneous aerogel.

### Example 5

The aerogel is obtained as describes in example 1, 2, 3 or 4. The aerogel which comprises one or more enzyme(s) as well as the aerogel which comprises one or more enzyme(s) and one or more other bioactive agent(s) are according to the present invention. The one or more enzymes and/or proteins are modified on amino groups (NH2) and/or on thiol groups (SH) and/or on OH groups. With succinimid, glutaraldehyde or isocyanat groups a desired group can be fixed to the protein and/or enzyme. In one preferred embodiment polyethylenglycol (PEG) is used as substituent. An alternative to PEG is PEG linked to long alkyl chain and/or acryl and/or vinyl groups.

### Example 6

Esperase has been encapsulated in an aerogel. Tetrapeptide staining has demonstrated that Esperase retain its actitivy after encapsulation into the aerogel. Heating of the aerogel encapsulating Esperase to 80°C for 24 hour shows that the enzyme activity is retained after the heating. Heating of the aerogel encapsulating Esperase in water to 80°C for 24 hour shows that the enzyme activity is decreased. This demonstrate that Esperase is stabilised by encapsulation into the aerogel.

### Example 7

Two different self-polishing solvent based paints have been formulated with aerogels containing two different proteases. Panels with the paints have been immersed in sea water in Elsinore Harbour, Denmark, for a period of 6 months (May- October inclusive). No fouling was observed after the period, or a very thin layer of algal, slime was detected compared to controls.

### Example 8

Test fields were applied with same kind of formulation containing aerogel and protease on both sides of the rudder of a sailing boat based in Ishøj Harbour, Denmark. Inspection after the sailing season 6 months later showed very thin algal slime and of the same fouling degree as the conventional cupper based antifouling paint.

### Example 9

The aerogel is obtained as in examples 1,2,3 and 4 wherein only those aerogels comprising at least one enzyme are according to the invention. The one or more enzymes/ and or proteins are modified on amino groups (NH2) and/or on thiol groups (SH) and or on hydroxyl groups (OH). The substituent include a s SiR1 R2R3R4 group where R1 links the Si to the enzyme and R2 R3 and R4 are chosen among Cl,Br,I and OR5 where R5 is an alkyl group. A preferred embodiment R5= CH3 and R2=R3=R4= OR5. Hereby the enzyme can be chemically linked into the gel network modifying its lifetime on gel surfaces exposed to water.

### Example 10

| Example of preparations | Silicagel, weight-% | Enzyme preparation, weight-% |
|---|---|---|
| AP29 | 83,9 | 16,1 |
| AP34 | 61,1 | 38,9 |
| AP35 | 44,9 | 55,1 |
| AP32 | 25,4 | 74,6 |

### Example 11

Compositions according to the invention and not according to the invention were applied to well-defined panels on a raft and the raft was submerged in water at Elsinore harbour, Denmark. The raft was submerged for a period of 6 months before the results were evaluated. Also included on the raft were panels with conventional paints and controls. The panels of the reafts were treated as indicated in the following table:

### Panels of the test raft

| Panel 1 Sigma | Panel 2 MPT7-Z-AP | Panel 3 MPT3-Z-AE | Panel 4 MPT7-Z-AE |
|---|---|---|---|
| Panel 5 | Panel 6 | Panel 7 | Panel 8 |
| MPT7-Z-A | MPT3-Z-AE | MPT3-ZS-AP | SE-ZS-AE |
| Panel 9 | Panel 10 | Panel 11 | Panel 12 |
| MPT3-Z-A | MPT7-Z-AP | MPT7-Z-AE | Mille Xtra |

The compositions applied to the raft as indicated in the above table contained the following components:
MPT3: Base paint (the figure "3" refers to the amount of rosin in relation to harpiks extender)
MPT7: Base paint (the figure "7" refers to the amount of rosin in relation to harpiks extender)
SE: Solvent epoxy paint
Z: Zink
S: Sulphide
A: Aerogel
P: Polarzyme
E: Esperase
Sigma: Positive control (conventional antifouling paint, Sigma cupper based product + a biocide)
Mille Xtra: Positive control (conventional antifouling paint, Hempel self-polishing cupper based product)

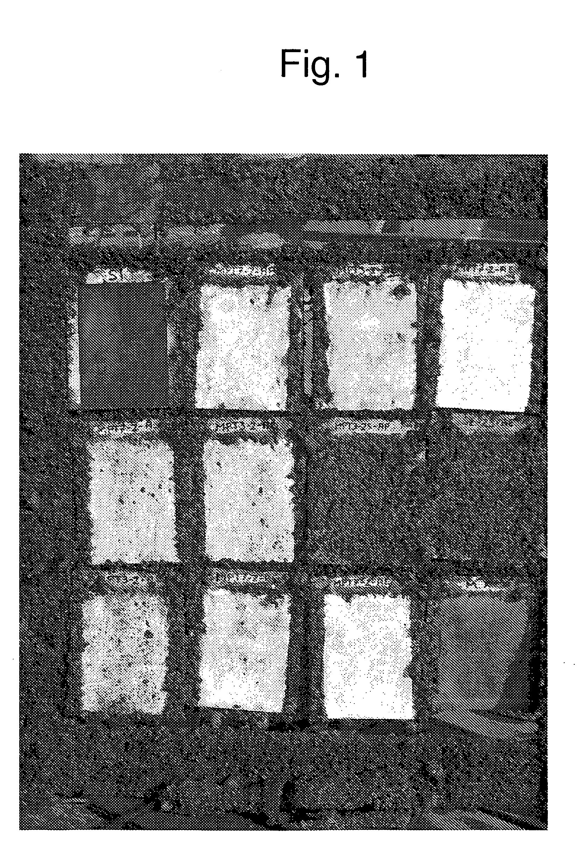

At the end of the test period of 6 months, the fouling of each panel on the raft was evaluated by using a score from 0 to 5, where 0 is no fouling growth and 5 is complete coverage of the panel by fouling growth. The results were as indicated in the table below. A picture of the raft after the experiment is included as fig. 1. The quantifications of the below table were based on the picture of fig. 1.

### Quantification of the growth on the panels of the test raft

| Panel 1 | Panel 2 | Panel 3 | Panel 4 |
|---|---|---|---|
| 0-1 | 1-2 | 2 | 0 |

| Panel 5 | Panel 6 | Panel 7 | Panel 8 |
|---|---|---|---|
| 2-3 | 2 | 5 | 5 |

| Panel 9 | Panel 10 | Panel 11 | Panel 12 |
|---|---|---|---|
| 3 | 1-2 | 0-1 | 2 |

A conclusion of the test was that the MPT7-Z-AE panels (panels 4 and 11) were least fouled at the end of the test. These panels had been treated with the base paint MPT7 comprising aerogel, esperase enzyme and zinc. The MPT7-Z-AE panels proved to be at least as good or possibly even slightly better than the conventional Sigma cupper-based paint. This result was unexpectedly positive and supports the advantageous effect of the present invention. Also, it was evident that the compositions comprising both aerogel and enzyme (panels 2, 3, 4, 6, 10, and 11) were generally better at preventing fouling than the panels comprising aerogel without enzyme (panels 5 and 9).

### Example 12

The rudder from the yacht Erica was painted with 9 different coatings as indicated in the table herein below. The results were evaluated after a sailing a season (5 Months) in Danish seawater. The evaluation is a quantification of the growth using a score from 0 to 5, where 0 is no fouling growth and 5 is complete coverage of the panel by fouling growth.

| **Starbord side of rudder** | | | | **Port side of rudder** | | |
|---|---|---|---|---|---|---|
| Torm G | Solv 6 | Solv 10 | | Solv 14 | Solv 17 | Solv 4 |
| Solv 7 | Solv 9 | Solv 17 | | Solv 7 | Solv 9 | Solv 8 |
| Sovl 8 | Solv 4 | Solv 14 | | Solv 10 | Torm G | Solv 6 |

| **Evaluation of the test** | | | | | | |
|---|---|---|---|---|---|---|
| 1 | 1-2 | 4 | | 4 | 5 | 1 |
| 5 | 5 | 5 | | 5 | 5 | 1 |
| 2-3 | 3 | 5 | | 5 | 1 | 2 |

The compositions tested were:

| | **Aerogel with esperase enzyme** | **PVC Activ** | **Rosin/retarder** | |
|---|---|---|---|---|
| **SOLV 4** | 0.8 | 32.8 | 03 : 01 | |
| **SOLV 6** | 0.2 | 16.3 | 7.5 : 1 | |
| **SOLV 7** | 1.1 | 16.4 | 03 : 01 | |
| **SOLV 8** | 0.2 freeze-dried esperase | 32.7 | 03 : 01 | |
| **SOLV 9** | 0.4 | 15.2 | 7.6 : 1 | Comprises silver |
| **SOLV 10** | 0.8 | 16.5 | 03 : 01 | Comprises silver |
| **SOLV 14** | 0.6 | 21.4 | 3.9 : 1 | |
| **SOLV 17** | 0.8 | 18.9 | 3.4 : 1 | |
| **TORM G** | 0.4 | 27.4 | 5.4 : 1 | |

## Claims

1. An anti-fouling composition for reduction or prevention of fouling of a surface coated with said composition, the anti-fouling composition comprising one or more aerogel(s) and one or more bioactive agent(s) encapsulated into the aerogel(s), wherein the one or more bioactive agent(s) comprises one or more enzyme(s).

2. The anti-fouling composition comprising one or more aerogel(s) according to claim 1, wherein the one or more aerogel(s) is selected from one or more organic aerogel(s), one or more in-organic aerogel(s), one or more silica aerogel(s), one or more carbon aerogel(s), or one or more metal aerogel(s).

3. The anti-fouling composition comprising one or more aerogel(s) according to claim 1, wherein the one or more aerogel(s) has a density in the range from 0.05 to 1.0 g/cm³ such as 0.05 g/cm³ to 0.45 g/cm³, for example 0.05 g/cm³ to 0.4 g/cm³, such as 0.05 g/cm³ to 0.35 g/cm³, for example 0.05 g/cm³ to 0.3 g/cm³, such as 0.05 g/cm³ to 0.35 g/cm³, for example 0.05 g/cm³ to 0.3 g/cm³, such as 0.05 g/cm³ to 0.25 g/cm³, for example 0.05 g/cm³ to 0.2 g/cm³, such as 0.05 g/cm³ to 0.15 g/cm³, for example 0.05 g/cm³ to 0.1 g/cm³, such as 0.05 g/cm³ to 0.5 g/cm³, for example 0.1 g/cm³ to 0.5 g/cm³, such as 0.15 g/cm² to 0.5 g/cm³, for example 0.2 g/cm³ to 0.5 g/cm³, such as 0.25 g/cm³ to 0.5 g/cm³, for example 0.3 g/cm³ to 0.5 g/cm³, such as 0.35 g/cm³ to 0.5 g/cm³, for example 0.4 g/cm³ to 0.5 g/cm³, such as 0.45 g/cm³ to 0.5 g/cm³, for example 0.05 g/cm³ to 1.0 g/cm³, such as 0.05 g/cm³ to 0.95 g/cm³, for example 0.05 g/cm³ to 0.9 g/cm³, such as 0.05 g/cm³ to 0.85 g/cm³, for example 0.05 g/cm³ to 0.8 g/cm³, such as 0.05 g/cm³ to 0.75 g/cm³, for example 0.05 g/cm³ to 0.7 g/cm³, such as 0.05 g/cm³ to 0.65 g/cm³, for example 0.05 g/cm³ to 0.6 g/cm³, such as 0.05 g/cm³ to 0.55 g/cm³, for example 0.05 g/cm³ to 0.5 g/cm³, such as 0.05 g/cm³ to 0.45 g/cm³, for example 0.05 g/cm³ to 0.4 g/cm³, such as 0.05 g/cm³ to 0.35 g/cm³, for example 0.05 g/cm³ to 0.3 g/cm³, such as 0.05 g/cm³ to 0.25 g/cm³, for example 0.05 g/cm³ to 0.2 g/cm³, such as 0.05 g/cm³ to 0.15 g/cm³, for example 0.05 g/cm³ to 0.1 g/cm³, such as 0.1 g/cm³ to 1.0 g/cm³, for example 0.15 g/cm³ to 1.0 g/cm³, such as 0.2 g/cm³ to 1.0 g/cm³, for example 0.25 g/cm³ to 1.0 g/cm³, such as 0.3 g/cm³ to 1.0 g/cm³, for example 0.35 g/cm³ to 1.0 g/cm³, such as 0.4 g/cm³ to 1.0 g/cm³, for example 0.45 g/cm³ to 1.0 g/cm³, such as 0.5 g/cm³ to 1.0 g/cm³, for example 0.55 g/cm³ to 1.0 g/cm³, such as 0.6 g/cm³ to 1.0 g/cm³, for example 0.65 g/cm³ to 1.0 g/cm³, such as 0.7 g/cm³ to 1.0 g/cm³, for example 0.75 g/cm³ to 1.0 g/cm³, such as 0.8 g/cm³ to 1.0 g/cm³, for example 0.85 g/cm³ to 1.0 g/cm³, such as 0.9 g/cm³ to 1.0 g/cm³, for example 0.95 g/cm³ to 1.0 g/cm³, such as 0.15 g/cm³ to 0.3 g/cm³, for example 0.16 g/cm³ to 0.3 g/cm³, such as 0.17 g/cm³ to 0.3 g/cm³, for example 0.18 g/cm³ to 0.3 g/cm³, such as 0.19 g/cm³ to 0.3 g/cm³, for example 0.20 g/cm³ to 0.3 g/cm³, such as 0.21 g/cm³ to 0.3 g/cm³, for example 0.22g/cm³ to 0.3 g/cm³, such as 0.23 g/cm³ to 0.3 g/cm³, for example 0.24 g/cm² to 0.3 g/cm³, such as 0.25 g/cm³ to 0.3 g/cm³, for example 0.26 g/cm³ to 0.3 g/cm³, such as 0.27 g/cm³ to 0.3 g/cm³, for example 0.28 g/cm³ to 0.3 g/cm³, such as 0.29 g/cm³ to 0.3 g/cm³, such as 0.15 g/cm³ to 0.29 g/cm³, such as 0.15 g/cm³ to 0,28 g/cm³, such as 0.15 g/cm³ to 0.27 g/cm³, such as 0.15 g/cm³ to 0.26 g/cm³, such as 0.15 g/cm³ to 0.25 g/cm³, such as 0.15 g/cm³ to 0.24 g/cm³, such as 0.15 g/cm³ to 0.23 g/cm³, such as 0.15 g/cm³ to 0.22 g/cm³, such as 0.15 g/cm³ to 0.21 g/cm³, such as 0.15 g/cm³ to 0.20 g/cm³, such as 0.15 g/cm³ to 0.19 g/cm³, such as 0.15 g/cm³ to 0.18 g/cm³, such as 0.15 g/cm³ to 0.17 g/cm³, such as 0.15 g/cm³ to 0.16 g/cm³.

4. The anti-fouling composition comprising one or more aerogel(s) according to claim 1, wherein the one or more aerogel(s) has a surface area in the range from 500 m²/g to 2000 m²/g such as from 500 m²/g to 1950 m²/g, for example from 500 m²/g to 1900 m²/g, such as from 500 m²/g to 1850 m²/g, for example from 500 m²/g to 1800 m²/g, such as from 500 m²/g to 1750 m²/g, for example from 500 m²/g to 1700 m²/g, such as from 500 m²/g to 1650 m²/g, for example from 500 m²/g to 1600 m²/g, such as from 500 m²/g to 1550 m²/g, for example from 500 m²/g to 1500 m²/g, such as from 500 m²/g to 1450 m²/g, for example from 500 m²/g to 1400 m²/g, such as from 500 m²/g to 1350 m²/g, for example from 500 m²/g to 1300 m²/g, such as from 500 m²/g to 1250 m²/g, for example from 500 m²/g to 1200 m²/g, such as from 500 m²/g to 1150 m²/g, for example from 500 m²/g to 1100 m²/g, such as from 500 m²/g to 1050 m²/g, for example from 500 m²/g to 1000 m²/g, such as from 500 m²/g to 950 m²/g, for example from 500 m²/g to 900 m²/g, such as from 500 m²/g to 850 m²/g, for example from 500 m²/g to 800 m²/g, such as from 500 m²/g to 750 m²/g, for example from 500 m²/g to 700 m²/g, such as from 500 m²/g to 650 m²/g, for example from 500 m²/g to 600 m²/g, such as from 500 m²/g to 550 m²/g, for example from 500 m²/g to 2000 m²/g, such as from 550 m²/g to 2000 m²/g, for example from 600 m²/g to 2000 m²/g, such as from 650 m²/g to 2000 m²/g, for example from 700 m²/g to 2000 m²/g, such as from 750 m²/g to 2000 m²/g, for example from 800 m²/g to 2000 m²/g, such as from 850 m²/g to 2000 m²/g, for example from 900 m²/g to 2000 m²/g, such as from 950 m²/g to 2000 m²/g, for example from 1000 m²/g to 2000 m²/g, such as from 1050 m²/g to 2000 m²/g, for example from 1100 m²/g to 2000 m²/g, such as from 1150 m²/g to 2000 m²/g, for example from 1200 m²/g to 2000 m²/g, such as from 1250 m²/g to 2000 m²/g, for example from 1300 m²/g to 2000 m²/g, such as from 1350 m²/g to 2000 m²/g, for example from 1400 m²/g to 2000 m²/g, such as from 1450 m²/g to 2000 m²/g, for example from 1500 m²/g to 2000 m²/g, such as from 1550 m²/g to 2000 m²/g, for example from 1600 m²/g to 2000 m²/g, such as from 1650 m²/g to 2000 m²/g, for example from 1700 m²/g to 2000 m²/g, such as from 1750 m²/g to 2000 m²/g, for example from 1800 m²/g to 2000 m²/g, such as from 1850 m²/g to 2000 m²/g, for example from 1900 m²/g to 2000 m²/g, such as from 1950 m²/g to 2000 m²/g, for example from 800 m²/g to 1500 m²/g, such as from 850 m²/g to 1500 m²/g, for example from 900 m²/g to 1500 m²/g, such as from 950 m²/g to 1500 m²/g, for example from 1000 m²/g to 1500 m²/g, such as from 1050 m²/g to 1500 m²/g, for example from 1100 m²/g to 1500 m²/g, such as from 1150 m²/g to 1500 m²/g, for example from 1200 m²/g to 1500 m²/g, such as from 1250 m²/g to 1500 m²/g, for example from 1300 m²/g to 1500 m²/g, such as from 1350 m²/g to 1500 m²/g, for example from 1400 m²/g to 1500 m²/g, such as from 1450 m²/g to 1500 m²/g, for example from 800 m²/g to 1450 m²/g, such as from 800 m²/g to 1400 m²/g, for example from 800 m²/g to 1350 m²/g, such as from 800 m²/g to 1300 m²/g, for example from 800 m²/g to 1250 m²/g, such as from 800 m²/g to 1200 m²/g, for example from 800 m²/g to 1150 m²/g, such as from 800 m²/g to 1100 m²/g, for example from 800 m²/g to 1050 m²/g, such as from 800 m²/g to 1000 m²/g, for example from 800 m²/g to 950 m²/g, such as from 800 m²/g to 900 m²/g, for example from 800 m²/g to 850 m²/g.

5. The anti-fouling composition comprising one or more aerogel(s) according to claim 1, wherein the one or more aerogel(s) has a pore size in the range of from 1 to 25 nm such as from 1 to 24 nm, for example from 1 to 22 nm, such as from 1 to 20 nm, for example from 1 to 18 nm, such as from 1 to 16 nm, for example from 1 to 14 nm, such as from 1 to 12 nm, for example from 1 to 10 nm, such as from 1 to 8 nm, for example from 1 to 6 nm, such as from 1 to 4 nm, for example from 1 to 2 nm, such as from 2 to 25 nm, for example from 4 to 25 nm, such as from 6 to 25 nm, for example from 8 to 25 nm, such as from 10 to 25 nm, for example from 12 to 25 nm, such as from 14 to 25 nm, for example from 16 to 25 nm, such as from 18 to 25 nm, for example from 20 to 25 nm, such as from 22 to 25 nm, for example from 24 to 25 nm, such as from 1 to 5 nm, for example from 5 to 10 nm, such as from 10 to 15 nm, for example from 15 to 20 nm, such as from 20 to 25 nm, for example from 2 to 10 nm, such as from 2 to 9 nm, for example from 2 to 8 nm, such as from 2 to 7 nm, for example from 2 to 6 nm, such as from 2 to 5 nm, for example from 2 to 4 nm, such as from 2 to 3 nm, for example from 3 to 10 nm, such as from 3 to 9 nm, for example from 3 to 8 nm, such as from 3 to 7 nm, for example from 3 to 6 nm, such as from 3 to 5 nm, for example from 3 to 4 nm, such as from 4 to 10 nm, for example from 4 to 9 nm, such as from 4 to 8 nm, for example from 4 to 7 nm, such as from 4 to 6 nm, for example from 4 to 5 nm, such as from 5 to 9 nm, for example from 5 to 8 nm, such as from 5 to 7 nm, for example from 5 to 6 nm, such as from 6 to 10 nm, for example from 6 to 9 nm, such as from 6 to 8 nm, for example from 6 to 7 nm, such as from 7 to 10 nm, for example from 7 to 9 nm, such as from 7 to 8 nm, for example from 8 to 10 nm, such as from 8 to 9 nm, for example from 9 to 10 nm.

6. The anti-fouling composition comprising one or more aerogel(s) according to claim 1, wherein the one or more bioactive agent(s) are released from the one or more aerogel(s) over time.

7. The anti-fouling composition comprislng one or more aerogel(s) according to claim 1, wherein the one or more enzymes comprises one or more hemicellulolytically active enzyme(s), such as xylanase, one or more amylolytically active enzyme(s), such as amylase, or one or more cellulolytically active enzyme(s) and/or one or more hydrolytic enzymes and/or one or more lipase(s), such as triacylglycerol lipase and lipoprotein lipase.

8. The anti-fouling composition comprising one or more aerogel(s) according to claim 1, wherein the one or more bioactive agents) comprises one or more endopeptidase(s), preferably comprising a Subtilisin (EC 3.4.21.62).

9. The anti-fouling composition comprising one or more aerogel(s) according to claim 7, wherein the one or more hemicellulolytically active enzyme(s) is selected from the group consisting of Endo-1,4-beta-xylanase (E.C. 3.2.1.8), Xylan endo-1,3-beta-xylosidase (E.C. 3.2.1.32). Glucuronoarabinoxylan endo-1,4-beta-xylanase (E.C. 3.2.1.136), Beta-mannosidase (E.C. 3.2.1.25), Mannan endo-1,4-beta-mannosidase (E.C. 3.2.1.78) and Mannan endo-1,6-beta-mannosidase (E.C. 3.2.1.101).

10. The anti-fouling composition comprising one or more aerogel(s) according to any of the preceding claims, wherein the composition further comprises an esterase, a lipase and/or a protease, preferably a protease of the subtilisin type.

11. The anti-fouling composition comprising one or more aerogel(s) according to claim 1, wherein the one or more enzyme(s) are functionalized to be actively incorporated into the three-dimensional structure of the one or more aerogel(s),

12. The anti-fouling composition comprising one or more aerogel(s) according to claim 1, wherein the one or more enzyme(s) are encapsulated in the one or more aerogels together with one or more other bioactive agent(s).

13. The anti-fouling composition comprising one or more aerogel(s) according to any of the preceding claims, wherein the composition further comprises a rosin.

14. A coating composition comprising the anti-fouling composition comprising one or more aerogels according to any of the claims 1-13 and a carrier.

15. The coating composition according to claim 14, wherein the coating composition comprises at least one of one or more pigment(s), one or more binder(s), one or more vehicle(s).

16. A method for preventing or reducing fouling of a surface, said method comprising the steps of contacting the surface with a composition according to any of claims 1 to 15 with an effective amount of said composition or coating composition or hygienic composition, wherein said contacting results in preventing or reducing fouling of said surface.

17. Method for preparing the composition according to any of claims 1 to 15, said method comprising the steps of providing at least one pigment and at least one enzyme capable of acting on a compound, wherein said action results in the formation of an antifouling species comprising an antifouling activity, wherein said compound does not form part of said composition, further providing a carrier for said at least one enzyme, and forming said composition by contacting said at least one enzyme with said carrier.

## Patentansprüche

1. Antifouling-Zusammensetzung zur Reduktion oder Verhinderung von Fouling einer mit der Zusammensetzung beschichteten Oberfläche, wobei die Antifouling-Zusammensetzung ein oder mehrere Aerogel(e) und ein oder mehrere in das/die Aerogel(e) verkapselte(s) bioaktive(s) Mittel umfasst, wobei das eine oder die mehreren bioaktive(n) Mittel ein oder mehrere Enzym(e) umfasst bzw. umfassen.

2. Antifouling-Zusammensetzung umfassend ein oder mehrere Aerogel(e) nach Anspruch 1, wobei das eine oder die mehreren Aerogel(e) ausgewählt ist bzw. sind aus einem oder mehreren organischen Aerogel(en), einem oder mehreren anorganischen Aerogel(en), einem oder mehreren Silica-Aerogel(en), einem oder mehreren Kohlenstoff-Aerogel(en) oder einem oder mehreren Metall-Aerogel(en).

3. Antifouling-Zusammensetzung umfassend ein oder mehrere Aerogel(e) nach Anspruch 1, wobei das eine oder die mehreren Aerogel(e) eine Dichte in dem Bereich von 0,05 bis 1,0 g/cm³, wie etwa 0,05 g/cm³ bis 0,45 g/cm³, zum Beispiel 0,05 g/cm³ bis 0,4 g/cm³, wie zum Beispiel 0,05 g/cm³ bis 0,35 g/cm³, zum Beispiel 0,05 g/cm³ bis 0,3 g/cm³, wie zum Beispiel 0,05 g/cm³ bis 0,35 g/cm³, zum Beispiel 0,05 g/cm³ bis 0,3 g/cm³, wie zum Beispiel 0,05 g/cm³ bis 0,25 g/cm³, zum Beispiel 0,05 g/cm³ bis 0,2 g/cm³, wie zum Beispiel 0,05 g/cm³ bis 0,15 g/cm³, zum Beispiel 0,05 g/cm³ bis 0,1 g/cm³, wie zum Beispiel 0,05 g/cm³ bis 0,5 g/cm³, zum Beispiel 0,1 g/cm³ bis 0,5 g/cm³, wie zum Beispiel 0,15 g/cm³ bis 0,5 g/cm³, zum Beispiel 0,2 g/cm³ bis 0,5 g/cm³, wie zum Beispiel 0,25 g/cm³ bis 0,5 g/cm³, zum Beispiel 0,3 g/cm³ bis 0,5 g/cm³, wie zum Beispiel 0,35 g/cm³ bis 0,5 g/cm³, zum Beispiel 0,4 g/cm³ bis 0,5 g/cm³, wie zum Beispiel 0,45 g/cm³ bis 0,5 g/cm³, zum Beispiel 0,05 g/cm³ bis 1,0 g/cm³, wie zum Beispiel 0,05 g/cm³ bis 0,95 g/cm³, zum Beispiel 0,05 g/cm³ bis 0,9 g/cm³, wie zum Beispiel 0,05 g/cm³ bis 0,85 g/cm³, zum Beispiel 0,05 g/cm³ bis 0,8 g/cm³, wie zum Beispiel 0,05 g/cm³ bis 0,75 g/cm³, zum Beispiel 0,05 g/cm³ bis 0,7 g/cm³, wie zum Beispiel 0,05 g/cm³ bis 0,65 g/cm³, zum Beispiel 0,05 g/cm³ bis 0,6 g/cm³, wie zum Beispiel 0,05 g/cm³ bis 0,55 g/cm³, zum Beispiel 0,05 g/cm³ bis 0,5 g/cm³, wie zum Beispiel 0,05 g/cm³ bis 0,45 g/cm³, zum Beispiel 0,05 g/cm³ bis 0,4 g/cm³, wie zum Beispiel 0,05 g/cm³ bis 0,35 g/cm³, zum Beispiel 0,05 g/cm³ bis 0,3 g/cm³, wie zum Beispiel 0,05 g/cm³ bis 0,25 g/cm³, zum Beispiel 0,05 g/cm³ bis 0,2 g/cm³, wie zum Beispiel 0,05 g/cm³ bis 0,15 g/cm³, zum Beispiel 0,05 g/cm³ bis 0,1 g/cm³, wie zum Beispiel 0,1 g/cm³ bis 1,0 g/cm³, zum Beispiel 0,15 g/cm³ bis 1,0 g/cm³, wie zum Beispiel 0,2 g/cm³ bis 1,0 g/cm³, zum Beispiel 0,25 g/cm³ bis 1,0 g/cm³, wie zum Beispiel 0,3 g/cm³ bis 1,0 g/cm³, zum Beispiel 0,35 g/cm³ bis 1,0 g/cm³, wie zum Beispiel 0,4 g/cm³ bis 1,0 g/cm³, zum Beispiel 0,45 g/cm³ bis 1,0 g/cm³, wie zum Beispiel 0,5 g/cm³ bis 1,0 g/cm³, zum Beispiel 0,55 g/cm³ bis 1,0 g/cm³, wie zum Beispiel 0,6 g/cm³ bis 1,0 g/cm³, zum Beispiel 0,65 g/cm³ bis 1,0 g/cm³, wie zum Beispiel 0,7 g/cm³ bis 1,0 g/cm³, zum Beispiel 0,75 g/cm³ bis 1,0 g/cm³, wie zum Beispiel 0,8 g/cm³ bis 1,0 g/cm³, zum Beispiel 0,85 g/cm³ bis 1,0 g/cm³, wie zum Beispiel 0,9 g/cm³ bis 1,0 g/cm³, zum Beispiel 0,95 g/cm³ bis 1,0 g/cm³, wie zum Beispiel 0,15 g/cm³ bis 0,3 g/cm³, zum Beispiel 0,16 g/cm³ bis 0,3 g/cm³, wie zum Beispiel 0,17 g/cm³ bis 0,3 g/cm³, zum Beispiel 0,18 g/cm³ bis 0,3 g/cm³, wie zum Beispiel 0,19 g/cm³ bis 0,3 g/cm³, zum Beispiel 0,20 g/cm³ bis 0,3 g/cm³, wie zum Beispiel 0,21 g/cm³ bis 0,3 g/cm³, zum Beispiel 0,22 g/cm³ bis 0,3 g/cm³, wie zum Beispiel 0,23 g/cm³ bis 0,3 g/cm³, zum Beispiel 0,24 g/cm³ bis 0,3 g/cm³, wie zum Beispiel 0,25 g/cm³ bis 0,3 g/cm³, zum Beispiel 0,26 g/cm³ bis 0,3 g/cm³, wie zum Beispiel 0,27 g/cm³ bis 0,3 g/cm³, zum Beispiel 0,28 g/cm³ bis 0,3 g/cm³, wie zum Beispiel 0,29 g/cm³ bis 0,3 g/cm³, wie zum Beispiel 0,15 g/cm³ bis 0,29 g/cm³, wie zum Beispiel 0,15 g/cm³ bis 0,28 g/cm³, wie zum Beispiel 0,15 g/cm³ bis 0,27 g/cm³, wie zum Beispiel 0,15 g/cm³ bis 0,26 g/cm³, wie zum Beispiel 0,15 g/cm³ bis 0,25 g/cm³, wie zum Beispiel 0,15 g/cm³ bis 0,24 g/cm³, wie zum Beispiel 0,15 g/cm³ bis 0,23 g/cm³, wie zum Beispiel 0,15 g/cm³ bis 0,22 g/cm³, wie zum Beispiel 0,15 g/cm³ bis 0,21 g/cm³, wie zum Beispiel 0,15 g/cm³ bis 0,20 g/cm³, wie zum Beispiel 0,15 g/cm³ bis 0,19 g/cm³, wie zum Beispiel 0,15 g/cm³ bis 0,18 g/cm³, wie zum Beispiel 0,15 g/cm³ bis 0,17 g/cm³, wie zum Beispiel 0,15 g/cm³ bis 0,16 g/cm³ aufweist bzw. aufweisen.

4. Antifouling-Zusammensetzung umfassend ein oder mehrere Aerogel(e) nach Anspruch 1, wobei das eine oder die mehreren Aerogel(e) eine Oberfläche in dem Bereich von 500 m²/g bis 2000 m²/g, wie zum Beispiel von 500 m²/g bis 1950 m²/g, zum Beispiel von 500 m²/g bis 1900 m²/g, wie zum Beispiel von 500 m²/g bis 1850 m²/g, zum Beispiel von 500 m²/g bis 1800 m²/g, wie zum Beispiel von 500 m²/g bis 1750 m²/g, zum Beispiel von 500 m²/g bis 1700 m²/g, wie zum Beispiel von 500 m²/g bis 1650 m²/g, zum Beispiel von 500 m²/g bis 1600 m²/g, wie zum Beispiel von 500 m²/g bis 1550 m²/g, zum Beispiel von 500 m²/g bis 1500 m²/g, wie zum Beispiel von 500 m²/g bis 1450 m²/g, zum Beispiel von 500 m²/g bis 1400 m²/g, wie zum Beispiel von 500 m²/g bis 1350 m²/g, zum Beispiel von 500 m²/g bis 1300 m²/g, wie zum Beispiel von 500 m²/g bis 1250 m²/g, zum Beispiel von 500 m²/g bis 1200 m²/g, wie zum Beispiel von 500 m²/g bis 1150 m²/g, zum Beispiel von 500 m²/g bis 1100 m²/g, wie zum Beispiel von 500 m²/g bis 1050 m²/g, zum Beispiel von 500 m²/g bis 1000 m²/g, wie zum Beispiel von 500 m²/g bis 950 m²/g, zum Beispiel von 500 m²/g bis 900 m²/g, wie zum Beispiel von 500 m²/g bis 850 m²/g, zum Beispiel von 500 m²/g bis 800 m²/g, wie zum Beispiel von 500 m²/g bis 750 m²/g, zum Beispiel von 500 m²/g bis 700 m²/g, wie zum Beispiel von 500 m²/g bis 650 m²/g, zum Beispiel von 500 m²/g bis 600 m²/g, wie zum Beispiel von 500 m²/g bis 550 m²/g, zum Beispiel von 500 m²/g bis 2000 m²/g, wie zum Beispiel von 550 m²/g bis 2000 m²/g, zum Beispiel von 600 m²/g bis 2000 m²/g, wie zum Beispiel von 650 m²/g bis 2000 m²/g, zum Beispiel von 700 m²/g bis 2000 m²/g, wie zum Beispiel von 750 m²/g bis 2000 m²/g, zum Beispiel von 800 m²/g bis 2000 m²/g, wie zum Beispiel von 850 m²/g bis 2000 m²/g, zum Beispiel von 900 m²/g bis 2000 m²/g, wie zum Beispiel von 950 m²/g bis 2000 m²/g, zum Beispiel von 1000 m²/g bis 2000 m²/g, wie zum Beispiel von 1050 m²/g bis 2000 m²/g, zum Beispiel von 1100 m²/g bis 2000 m²/g, wie zum Beispiel von 1150 m²/g bis 2000 m²/g, zum Beispiel von 1200 m²/g bis 2000 m²/g, wie zum Beispiel von 1250 m²/g bis 2000 m²/g, zum Beispiel von 1300 m²/g bis 2000 m²/g, wie zum Beispiel von 1350 m²/g bis 2000 m²/g, zum Beispiel von 1400 m²/g bis 2000 m²/g, wie zum Beispiel von 1450 m²/g bis 2000 m²/g, zum Beispiel von 1500 m²/g bis 2000 m²/g, wie zum Beispiel von 1550 m²/g bis 2000 m²/g, zum Beispiel von 1600 m²/g bis 2000 m²/g, wie zum Beispiel von 1650 m²/g bis 2000 m²/g, zum Beispiel von 1700 m²/g bis 2000 m²/g, wie zum Beispiel von 1750 m²/g bis 2000 m²/g, zum Beispiel von 1800 m²/g bis 2000 m²/g, wie zum Beispiel von 1850 m²/g bis 2000 m²/g, zum Beispiel von 1900 m²/g bis 2000 m²/g, wie zum Beispiel von 1950 m²/g bis 2000 m²/g, zum Beispiel von 800 m²/g bis 1500 m²/g, wie zum Beispiel von 850 m²/g bis 1500 m²/g, zum Beispiel von 900 m²/g bis 1500 m²/g, wie zum Beispiel von 950 m²/g bis 1500 m²/g, zum Beispiel von 1000 m²/g bis 1500 m²/g, wie zum Beispiel von 1050 m²/g bis 1500 m²/g, zum Beispiel von 1100 m²/g bis 1500 m²/g, wie zum Beispiel von 1150 m²/g bis 1500 m²/g, zum Beispiel von 1200 m²/g bis 1500 m²/g, wie zum Beispiel von 1250 m²/g bis 1500 m²/g, zum Beispiel von 1300 m²/g bis 1500 m²/g, wie zum Beispiel von 1350 m²/g bis 1500 m²/g, zum Beispiel von 1400 m²/g bis 1500 m²/g, wie zum Beispiel von 1450 m²/g bis 1500 m²/g, zum Beispiel von 800 m²/g bis 1450 m²/g, wie zum Beispiel von 800 m²/g bis 1400 m²/g, zum Beispiel von 800 m²/g bis 1350 m²/g, wie zum Beispiel von 800 m²/g bis 1300 m²/g, zum Beispiel von 800 m²/g bis 1250 m²/g, wie zum Beispiel von 800 m²/g bis 1200 m²/g, zum Beispiel von 800 m²/g bis 1150 m²/g, wie zum Beispiel von 800 m²/g bis 1100 m²/g, zum Beispiel von 800 m²/g bis 1050 m²/g, wie zum Beispiel von 800 m²/g bis 1000 m²/g, zum Beispiel von 800 m²/g bis 950 m²/g, wie zum Beispiel von 800 m²/g bis 900 m²/g, zum Beispiel von 800 m²/g bis 850 m²/g aufweist bzw. aufweisen.

5. Antifouling-Zusammensetzung umfassend ein oder mehrere Aerogel(e) nach Anspruch 1, wobei das eine oder die mehreren Aerogel(e) eine Porengröße in dem Bereich von 1 bis 25 nm, wie zum Beispiel von 1 bis 24 nm, zum Beispiel von 1 bis 22 nm, wie zum Beispiel von 1 bis 20 nm, zum Beispiel von 1 bis 18 nm, wie zum Beispiel von 1 bis 16 nm, zum Beispiel von 1 bis 14 nm, wie zum Beispiel von 1 bis 12 nm, zum Beispiel von 1 bis 10 nm, wie zum Beispiel von 1 bis 8 nm, zum Beispiel von 1 bis 6 nm, wie zum Beispiel von 1 bis 4 nm, zum Beispiel von 1 bis 2 nm, wie zum Beispiel von 2 bis 25 nm, zum Beispiel von 4 bis 25 nm, wie zum Beispiel von 6 bis 25 nm, zum Beispiel von 8 bis 25 nm, wie zum Beispiel von 10 bis 25 nm, zum Beispiel von 12 bis 25 nm, wie zum Beispiel von 14 bis 25 nm, zum Beispiel von 16 bis 25 nm, wie zum Beispiel von 18 bis 25 nm, zum Beispiel von 20 bis 25 nm, wie zum Beispiel von 22 bis 25 nm, zum Beispiel von 24 bis 25 nm, wie zum Beispiel von 1 bis 5 nm, zum Beispiel von 5 bis 10 nm, wie zum Beispiel von 10 bis 15 nm, zum Beispiel von 15 bis 20 nm, wie zum Beispiel von 20 bis 25 nm, zum Beispiel von 2 bis 10 nm, wie zum Beispiel von 2 bis 9 nm, zum Beispiel von 2 bis 8 nm, wie zum Beispiel von 2 bis 7 nm, zum Beispiel von 2 bis 6 nm, wie zum Beispiel von 2 bis 5 nm, zum Beispiel von 2 bis 4 nm, wie zum Beispiel von 2 bis 3 nm, zum Beispiel von 3 bis 10 nm, wie zum Beispiel von 3 bis 9 nm, zum Beispiel von 3 bis 8 nm, wie zum Beispiel von 3 bis 7 nm, zum Beispiel von 3 bis 6 nm, wie zum Beispiel von 3 bis 5 nm, zum Beispiel von 3 bis 4 nm, wie zum Beispiel von 4 bis 10 nm, zum Beispiel von 4 bis 9 nm, wie zum Beispiel von 4 bis 8 nm, zum Beispiel von 4 bis 7 nm, wie zum Beispiel von 4 bis 6 nm, zum Beispiel von 4 bis 5 nm, wie zum Beispiel von 5 bis 9 nm, zum Beispiel von 5 bis 8 nm, wie zum Beispiel von 5 bis 7 nm, zum Beispiel von 5 bis 6 nm, wie zum Beispiel von 6 bis 10 nm, zum Beispiel von 6 bis 9 nm, wie zum Beispiel von 6 bis 8 nm, zum Beispiel von 6 bis 7 nm, wie zum Beispiel von 7 bis 10 nm, zum Beispiel von 7 bis 9 nm, wie zum Beispiel von 7 bis 8 nm, zum Beispiel von 8 bis 10 nm, wie zum Beispiel von 8 bis 9 nm, zum Beispiel von 9 bis 10 nm aufweist bzw. aufweisen.

6. Antifouling-Zusammensetzung umfassend ein oder mehrere Aerogel(e) nach Anspruch 1, wobei das eine oder die mehreren bioaktive(n) Mittel mit der Zeit von dem einen oder den mehreren Aerogel(en) freigesetzt wird bzw. werden.

7. Antifouling-Zusammensetzung umfassend ein oder mehrere Aerogel(e) nach Anspruch 1, wobei das eine oder die mehreren Enzym(e) ein oder mehrere hemizellulolytisch aktive(s) Enzym(e), wie etwa Xylanase, oder ein oder mehrere amylolytisch aktive(s) Enzym(e), wie etwa Amylase, oder ein oder mehrere zellulolytisch aktive(s) Enzym(e) und/oder ein oder mehrere hydrolytische Enzyme und/oder eine oder mehrere Lipase(n), wie etwa Triacylglycerol-Lipase und Lipoprotein-Lipase umfasst bzw. umfassen.

8. Antifouling-Zusammensetzung umfassend ein oder mehrere Aerogel(e) nach Anspruch 1, wobei das eine oder die mehreren bioaktive(n) Mittel eine oder mehrere Endopeptidase(n) umfasst bzw. umfassen, vorzugsweise umfassend ein Subtilisin (EC 3.4.21.62).

9. Antifouling-Zusammensetzung umfassend ein oder mehrere Aerogel(e) nach Anspruch 7, wobei das eine oder die mehreren hemizellulolytisch aktive(n) Enzym(e) ausgewählt ist bzw. sind aus der Gruppe bestehend aus Endo-1,4-beta-xylanase (E.C. 3.2.1.8), Xylan-endo-1,3-beta-xylosidase (E.C. 3.2.1.32), Glucuronoarabinoxylan-endo-1,4-beta-xylanase (E.C. 3.2.1.136), Beta-mannosidase (E.C. 3.2.1.25), Mannan-endo-1,4-beta-mannosidase (E.C. 3.2.1.78) und Mannan-endo-1,6-beta-mannosidase (E.C. 3.2.1.101).

10. Antifouling-Zusammensetzung umfassend ein oder mehrere Aerogel(e) nach einem der vorhergehenden Ansprüche, wobei die Zusammensetzung des Weiteren eine Esterase, eine Lipase und/oder eine Protease, vorzugsweise eine Protease vom Subtilisin-Typ, umfasst.

11. Antifouling-Zusammensetzung umfassend ein oder mehrere Aerogel(e) nach Anspruch 1, wobei das eine oder die mehreren Enzym(e) funktionalisiert ist bzw. sind, um aktiv in die dreidimensionale Struktur des einen Aerogels oder der mehreren Aerogel eingebaut zu werden.

12. Antifouling-Zusammensetzung umfassend ein oder mehrere Aerogel(e) nach Anspruch 1, wobei das eine oder die mehreren Enzym(e) in dem einen oder den mehreren Aerogel(en) zusammen mit einem oder mehreren bioaktiven Mittel(n) verkapselt ist bzw. sind.

13. Antifouling-Zusammensetzung umfassend ein oder mehrere Aerogel(e) nach einem der vorhergehenden Ansprüche, wobei die Zusammensetzung des Weiteren ein Harz umfasst.

14. Beschichtungszusammensetzung umfassend die Antifouling-Zusammensetzung umfassend ein oder mehrere Aerogele nach einem der Ansprüche 1 bis 13 und einen Träger.

15. Beschichtungszusammensetzung nach Anspruch 14, wobei die Beschichtungszusammensetzung zumindest eines aus einem oder mehreren Pigment(en), einem oder mehreren Bindemittel(n) und einem oder mehreren Träger(n) umfasst.

16. Verfahren zur Verhinderung oder Reduktion von Fouling an einer Oberfläche, wobei das Verfahren die Schritte umfasst, dass die Oberfläche mit einer Zusammensetzung nach einem der Ansprüche 1 bis 15 mit einer effektiven Menge der Zusammensetzung oder Beschichtungszusammensetzung oder hygienischen Zusammensetzung in Kontakt gebracht wird, wobei der Kontakt zu einer Verhinderung oder Reduktion des Fouling an der Oberfläche führt.

17. Verfahren zur Herstellung der Zusammensetzung nach einem der Ansprüche 1 bis 15, wobei das Verfahren die Schritte umfasst, dass zumindest ein Pigment und zumindest ein Enzym, das auf eine Verbindung wirken kann, bereitgestellt werden, wobei die Wirkung in der Bildung einer Antifouling-Spezies mit einer Antifouling-Aktivität resultiert, wobei die Verbindung keinen Teil der Zusammensetzung bildet, des Weiteren den Schritt, dass ein Träger für das zumindest eine Enzym bereitgestellt wird, und dass die Zusammensetzung durch Inkontaktbringen des zumindest einen Enzyms mit dem Träger gebildet wird.

## Revendications

1. Composition antisalissure pour la réduction ou prévention de la salissure d'une surface revêtue avec ladite composition, la composition antisalissure comprenant un ou plusieurs aérogel(s) et un ou plusieurs agent(s) bioactif(s) encapsulé(s) dans le(s) aérogel(s), l'un ou les plusieurs agent(s) bioactif(s) comprenant une ou plusieurs enzyme(s).

2. Composition antisalissure comprenant un ou plusieurs aérogel(s) selon la revendication 1, dans laquelle l'un ou les plusieurs aérogel(s) est/sont choisi(s) parmi un ou plusieurs aérogel(s) organique(s), un ou plusieurs aérogel(s) inorganique(s), un ou plusieurs aérogel(s) de silice, un ou plusieurs aérogel(s) de carbone, ou un ou plusieurs aérogel(s) métallique(s).

3. Composition antisalissure comprenant un ou plusieurs aérogel(s) selon la revendication 1, dans laquelle l'un ou les plusieurs aérogel(s) présente(nt) une densité comprise entre 0,05 à 1,0 g/mol comme de 0,05 g/mol à 0,45 g/mol, par exemple 0,05 g/mol à 0,4 g/mol, comme de 0,05 g/mol à 0,35 g/mol, par exemple 0,05 g/mol à 0,3 g/mol, comme de 0,05 g/mol à 0,35 g/mol, par exemple 0,05 g/mol à 0,3 g/mol, comme de 0,05 g/mol à 0,25 g/mol, par exemple 0,05 g/mol à 0,2 g/mol, comme de 0,05 g/mol à 0,15 g/mol, par exemple 0,05 g/mol à 0,1 g/mol, comme de 0,05 g/mol à 0,5 g/mol, par exemple 0,1 g/mol à 0,5 g/mol, comme de 0,15 g/mol à 0,5 g/mol, par exemple 0,2 g/mol à 0,5 g/mol, comme de 0,25 g/mol à 0,5 g/mol, par exemple 0,3 g/mol à 0,5 g/mol, comme de 0,35 g/mol à 0,5 g/mol, par exemple 0,4 g/mol à 0,5 g/mol, comme de 0,45 g/mol à 0,5 g/mol, par exemple 0,05 g/mol à 1,0 g/mol, comme de 0,05 g/mol à 0,95 g/mol, par exemple 0,05 g/mol à 0,9 g/mol, comme de 0,05 g/mol à 0,85 g/mol, par exemple 0,05 g/mol à 0,8 g/mol, comme de 0,05 g/mol à 0,75 g/mol, par exemple 0,05 g/mol à 0,7 g/mol, comme de 0,05 g/mol à 0,65 g/mol, par exemple 0,05 g/mol à 0,6 g/mol, comme de 0,05 g/mol à 0,55 g/mol, par exemple 0,05 g/mol à 0,5 g/mol, comme de 0,05 g/mol à 0,45 g/mol, par exemple 0,05 g/mol à 0,4 g/mol, comme de 0,05 g/mol à 0,35 g/mol, par exemple 0,05 g/mol à 0,3 g/mol, comme de 0,05 g/mol à 0,25 g/mol, par exemple 0,05 g/mol à 0,2 g/mol, comme de 0,05 g/mol à 0,15 g/mol, par exemple 0,05 g/mol à 0,1 g/mol, comme de 0,1 g/mol à 1,0 g/mol, par exemple 0,15 g/mol à 1,0 g/mol, comme de 0,2 g/mol à 1,0 g/mol, par exemple 0,25 g/mol à 1,0 g/mol, comme de 0,3 g/mol à 1,0 g/mol, par exemple 0,35 g/mol à 1,0 g/mol, comme de 0,4 g/mol à 1,0 g/mol, par exemple 0,45 g/mol à 1,0 g/mol, comme de 0,5 g/mol à 1,0 g/mol, par exemple 0,55 g/mol à 1,0 g/mol, comme de 0,6 g/mol à 1,0 g/mol, par exemple 0,65 g/mol à 1,0 g/mol, comme de 0,7 g/mol à 1,0 g/mol, par exemple 0,75 g/mol à 1,0 g/mol, comme de 0,8 g/mol à 1,0 g/mol, par exemple 0,85 g/mol à 1,0 g/mol, comme de 0,9 g/mol à 1,0 g/mol, par exemple 0,95 g/mol à 1,0 g/mol, comme de 0,15 g/mol à 0,3 g/mol, par exemple 0,16 g/mol à 0,3 g/mol, comme de 0,17 g/mol à 0,3 g/mol, par exemple 0,18 g/mol à 0,3 g/mol, comme de 0,19 g/mol à 0,3 g/mol, par exemple 0,20 g/mol à 0,3 g/mol, comme de 0,21 g/mol à 0,3 g/mol, par exemple 0,22g/mol à 0,3 g/mol, comme de 0,23 g/mol à 0,3 g/mol, par exemple 0,24 g/mol à 0,3 g/mol, comme de 0,25 g/mol à 0,3 g/mol, par exemple 0,26 g/mol à 0,3 g/mol, comme de 0,27 g/mol à 0,3 g/mol, par exemple 0,28 g/mol à 0,3 g/mol, comme de 0,29 g/mol à 0,3 g/mol, comme de 0,15 g/mol à 0,29 g/mol, comme de 0,15 g/mol à 0,28 g/mol, comme de 0,15 g/mol à 0,27 g/mol, comme de 0,15 g/mol à 0,26 g/mol, comme de 0,15 g/mol à 0,25 g/mol, comme de 0,15 g/mol à 0,24 g/mol, comme de 0,15 g/mol à 0,23 g/mol, comme de 0,15 g/mol à 0,22 g/mol, comme de 0,15 g/mol à 0,21 g/mol, comme de 0,15 g/mol à 0,20 g/mol, comme de 0,15 g/mol à 0,19 g/mol, comme de 0,15 g/mol à 0,18 g/mol, comme de 0,15 g/mol à 0,17 g/mol, comme de 0,15 g/mol à 0,16 g/mol.

4. Composition antisalissure comprenant un ou plusieurs aérogel(s) selon la revendication 1, dans laquelle l'un ou les plusieurs aérogel(s) présente(nt) une surface comprise entre 500 m²/g à 2000 m²/g, comme de 500 m²/g à 1950 m²/g, par exemple de 500 m²/g à 1900 m²/g, comme de 500 m²/g à 1850 m²/g, par exemple de 500 m²/g à 1800 m²/g, comme de 500 m²/g à 1750 m²/g, par exemple de 500 m²/g à 1700 m²/g, comme de 500 m²/g à 1650 m²/g, par exemple de 500 m²/g à 1600 m²/g, comme de 500 m²/g à 1550 m²/g, par exemple de 500 m²/g à 1500 m²/g, comme de 500 m²/g à 1450 m²/g, par exemple de 500 m²/g à 1400 m²/g, comme de 500 m²/g à 1350 m²/g, par exemple de 500 m²/g à 1300 m²/g, comme de 500 m²/g à 1250 m²/g, par exemple de 500 m²/g à 1200 m²/g, comme de 500 m²/g à 1150 m²/g, par exemple de 500 m²/g à 1100 m²/g, comme de 500 m²/g à 1050 m²/g, par exemple de 500 m²/g à 1000 m²/g, comme de 500 m²/g à 950 m²/g, par exemple de 500 m²/g à 900 m²/g, comme de 500 m²/g à 850 m²/g, par exemple de 500 m²/g à 800 m²/g, comme de 500 m²/g à 750 m²/g, par exemple de 500 m²/g à 700 m²/g, comme de 500 m²/g à 650 m²/g, par exemple de 500 m²/g à 600 m²/g, comme de 500 m²/g à 550 m²/g, par exemple de 500 m²/g à 2000 m²/g, comme de 550 m²/g à 2000 m²/g, par exemple de 600 m²/g à 2000 m²/g, comme de 650 m²/g à 2000 m²/g, par exemple de 700 m²/g à 2000 m²/g, comme de 750 m²/g à 2000 m²/g, par exemple de 800 m²/g à 2000 m²/g, comme de 850 m²/g à 2000 m²/g, par exemple de 900 m²/g à 2000 m²/g, comme de 950 m²/g à 2000 m²/g, par exemple de 1000 m²/g à 2000 m²/g, comme de 1050 m²/g à 2000 m²/g, par exemple de 1 100 m²/g à 2000 m²/g, comme de 1 150 m²/g à 2000 m²/g, par exemple de 1200 m²/g à 2000 m²/g, comme de 1250 m²/g à 2000 m²/g, par exemple de 1300 m²/g à 2000 m²/g, comme de 1350 m²/g à 2000 m²/g, par exemple de 1400 m²/g à 2000 m²/g, comme de 1450 m²/g à 2000 m²/g, par exemple de 1500 m²/g à 2000 m²/g, comme de 1550 m²/g à 2000 m²/g, par exemple de 1600 m²/g à 2000 m²/g, comme de 1650 m²/g à 2000 m²/g, par exemple de 1700 m²/g à 2000 m²/g, comme de 1750 m²/g à 2000 m²/g, par exemple de 1800 m²/g à 2000 m²/g, comme de 1850 m²/g à 2000 m²/g, par exemple de 1900 m²/g à 2000 m²/g, comme de 1950 m²/g à 2000 m²/g, par exemple de 800 m²/g à 1500 m²/g, comme de 850 m²/g à 1500 m²/g, par exemple de 900 m²/g à 1500 m²/g, comme de 950 m²/g à 1500 m²/g, par exemple de 1000 m²/g à 1500 m²/g, comme de 1050 m²/g à 1500 m²/g, par exemple de 1 100 m²/g à 1500 m²/g, comme de 1150 m²/g à 1500 m²/g, par exemple de 1200 m²/g à 1500 m²/g, comme de 1250 m²/g à 1500 m²/g, par exemple de 1300 m²/g à 1500 m²/g, comme de 1350 m²/g à 1500 m²/g, par exemple de 1400 m²/g à 1500 m²/g, comme de 1450 m²/g à 1500 m²/g, par exemple de 800 m²/g à 1450 m²/g, comme de 800 m²/g à 1400 m²/g, par exemple de 800 m²/g à 1350 m²/g, comme de 800 m²/g à 1300 m²/g, par exemple de 800 m²/g à 1250 m²/g, comme de 800 m²/g à 1200 m²/g, par exemple de 800 m²/g à 1150 m²/g, comme de 800 m²/g à 1100 m²/g, par exemple de 800 m²/g à 1050 m²/g, comme de 800 m²/g à 1000 m²/g, par exemple de 800 m²/g à 950 m²/g, comme de 800 m²/g à 900 m²/g, par exemple de 800 m²/g à 850 m²/g.

5. Composition antisalissure comprenant un ou plusieurs aérogel(s) selon la revendication 1, dans laquelle l'un ou les plusieurs aérogel(s) présente(nt) une taille des pores comprise entre 1 à 25 nm, comme de 1 à 24 nm, par exemple de 1 à 22 nm, comme de 1 à 20 nm, par exemple de 1 à 18 nm, comme de 1 à 16 nm, par exemple de 1 à 14 nm, comme de 1 à 12 nm, par exemple de 1 à 10 nm, comme de 1 à 8 nm, par exemple de 1 à 6 nm, comme de 1 à 4 nm, par exemple de 1 à 2 nm, comme de 2 à 25 nm, par exemple de 4 à 25 nm, comme de 6 à 25 nm, par exemple de 8 à 25 nm, comme de 10 à 25 nm, par exemple de 12 à 25 nm, comme de 14 à 25 nm, par exemple de 16 à 25 nm, comme de 18 à 25 nm, par exemple de 20 à 25 nm, comme de 22 à 25 nm, par exemple de 24 à 25 nm, comme de 1 à 5 nm, par exemple de 5 à 10 nm, comme de 10 à 15 nm, par exemple de 15 à 20 nm, comme de 20 à 25 nm, par exemple de 2 à 10 nm, comme de 2 à 9 nm, par exemple de 2 à 8 nm, comme de 2 à 7 nm, par exemple de 2 à 6 nm, comme de 2 à 5 nm, par exemple de 2 à 4 nm, comme de 2 à 3 nm, par exemple de 3 à 10 nm, comme de 3 à 9 nm, par exemple de 3 à 8 nm, comme de 3 à 7 nm, par exemple de 3 à 6 nm, comme de 3 à 5 nm, par exemple de 3 à 4 nm, comme de 4 à 10 nm, par exemple de 4 à 9 nm, comme de 4 à 8 nm, par exemple de 4 à 7 nm, comme de 4 à 6 nm, par exemple de 4 à 5 nm, comme de 5 à 9 nm, par exemple de 5 à 8 nm, comme de 5 à 7 nm, par exemple de 5 à 6 nm, comme de 6 à 10 nm, par exemple de 6 à 9 nm, comme de 6 à 8 nm, par exemple de 6 à 7 nm, comme de 7 à 10 nm, par exemple de 7 à 9 nm, comme de 7 à 8 nm, par exemple de 8 à 10 nm, comme de 8 à 9 nm, par exemple de 9 à 10 nm.

6. Composition antisalissure comprenant un ou plusieurs aérogel(s) selon la revendication 1, dans laquelle l'un ou les plusieurs agent(s) bioactif(s) est/sont libéré(s) par l'un ou les plusieurs aérogel(s) avec le temps.

7. Composition antisalissure comprenant un ou plusieurs aérogel(s) selon la revendication 1, dans laquelle l'une ou les plusieurs enzyme(s) comprend/comprennent une ou plusieurs enzyme(s) avec activité hémicellulolytique, comme la xylanase, ou une ou plusieurs enzyme(s) avec activité amylolytique, comme l'amylase, ou une ou plusieurs enzyme(s) avec activité cellulolytique, et/ou une ou plusieurs enzyme(s) hydrolytique(s) et/ou une ou plusieurs lipase(s), comme la triacylglycérol-lipase et la lipase lipoprotéique.

8. Composition antisalissure comprenant un ou plusieurs aérogel(s) selon la revendication 1, dans laquelle l'un ou les plusieurs agent(s) bioactif(s) comprend/comprennent une ou plusieurs endopeptidase(s), de préférence comprenant un subtilisin (EC 3.4.21.62).

9. Composition antisalissure comprenant un ou plusieurs aérogel(s) selon la revendication 7, dans laquelle l'une ou les plusieurs enzyme(s) avec activité hémicellulolytique est/sont choisie(s) dans le groupe consistant en endo-1,4-bêta-xylanase (E.C. 3.2.1.8), xylane endo-1,3-bêta-xylosidase (E.C. 3.2.1.32), glucuronoarabinoxylane-endo-1,4-béta-xylanase (E.C. 3.2.1.136), bêta-mannosidase (E.C. 3.2.1.25), mannane endo-1,4-bêta-mannosidase (E.C. 3.2.1.78) et mannane endo-1,6-béta-mannosidase (E.C. 3.2.1.101).

10. Composition antisalissure comprenant un ou plusieurs aérogel(s) selon l'une quelconque des revendications précédentes, dans laquelle la composition en outre comprend une estérase, une lipase et/ou une protéase, de préférence une protéase de type subtilisin.

11. Composition antisalissure comprenant un ou plusieurs aérogel(s) selon la revendication 1, dans laquelle l'une ou les plusieurs enzyme(s) est/sont fonctionnalisée(s) afin de les activement incorporer dans la structure tridimensionnelle de l'un ou plusieurs aérogel(s).

12. Composition antisalissure comprenant un ou plusieurs aérogel(s) selon la revendication 1, dans laquelle l'une ou les plusieurs enzyme(s) est/sont encapsulée(s) dans l'un ou les plusieurs aérogel(s) ensemble avec un ou plusieurs autre(s) agent(s) bioactif(s).

13. Composition antisalissure comprenant un ou plusieurs aérogel(s) selon l'une quelconque des revendications précédentes, dans laquelle la composition en outre comprend une résine.

14. Composition de revêtement comprenant la composition antisalissure comprenant un ou plusieurs aérogels selon l'une quelconque des revendications 1 à 13 et un support.

15. Composition de revêtement selon la revendication 14, dans laquelle la composition de revêtement comprend au moins un d'un ou plusieurs pigment(s), un ou plusieurs liant(s), un ou plusieurs véhicule(s).

16. Procédé pour la prévention ou réduction de la salissure d'une surface, le procédé comprenant les étapes de contacter la surface avec une composition selon l'une quelconque des revendications 1 à 15 avec une quantité efficace de ladite composition ou composition de revêtement ou composition hygiénique, dans laquelle le contact résulte en la prévention ou réduction de la salissure de ladite surface.

17. Procédé pour la préparation de la composition selon l'une quelconque des revendications 1 à 15, ledit procédé comprenant les étapes de fournir au moins un pigment et au moins une enzyme capable d'agir sur un composé, dans lequel l'action résulte en la formation d'une espèce antisalissure comprenant une activité antisalissure, ledit composé ne faisant pas partie de ladite composition, en outre l'étape de fournir un support pour la au moins une enzyme, et former ladite composition en mettant en contact la au moins une enzyme avec ledit support.
